(19) Europäisches Patentamt
European Patent Office
Office européen des brevets

(11) EP 2 567 701 A2

(12) **DEMANDE DE BREVET EUROPEEN**

(43) Date de publication:
**13.03.2013 Bulletin 2013/11**

(21) Numéro de dépôt: **12180415.7**

(22) Date de dépôt: **25.06.2008**

(51) Int Cl.:
***A61K 31/585*** (2006.01) ***A61K 31/55*** (2006.01)
***A61K 31/401*** (2006.01) ***A61K 31/50*** (2006.01)
***A61K 31/501*** (2006.01) ***A61K 45/06*** (2006.01)
***A61P 9/04*** (2006.01)

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **28.04.2008 US 48419 P**
**26.06.2007 FR 0704584**

(62) Numéro(s) de document de la (des) demande(s) initiale(s) en application de l'article 76 CBE:
**08774270.6 / 2 170 349**

(71) Demandeur: **Ceva Sante Animale**
**33501 Libourne Cedex (FR)**

(72) Inventeurs:
- **Ovaert, Patricia**
**F-33500 Libourne (FR)**
- **Bernay, Florence**
**Leawood, KS Kansas 66224 (US)**
- **Guyonnet, Jerome**
**F-33440 Ambares (FR)**

(74) Mandataire: **Lecca, Patricia S.**
**Cabinet Lecca**
**21 rue de Fécamp**
**75012 Paris (FR)**

Remarques:
Cette demande a été déposée le 14-08-2012 comme demande divisionnaire de la demande mentionnée sous le code INID 62.

(54) **Traitement de l'insuffisance cardiaque chez les animaux mammifères non humains par un antagoniste de l'aldostérone.**

(57) L'invention concerne de nouvelles compositions comprenant un antagoniste de l'aldostérone selon une posologie particulière pour le traitement de l'insuffisance cardiaque des animaux mammifères non humains.

FIGURE 7

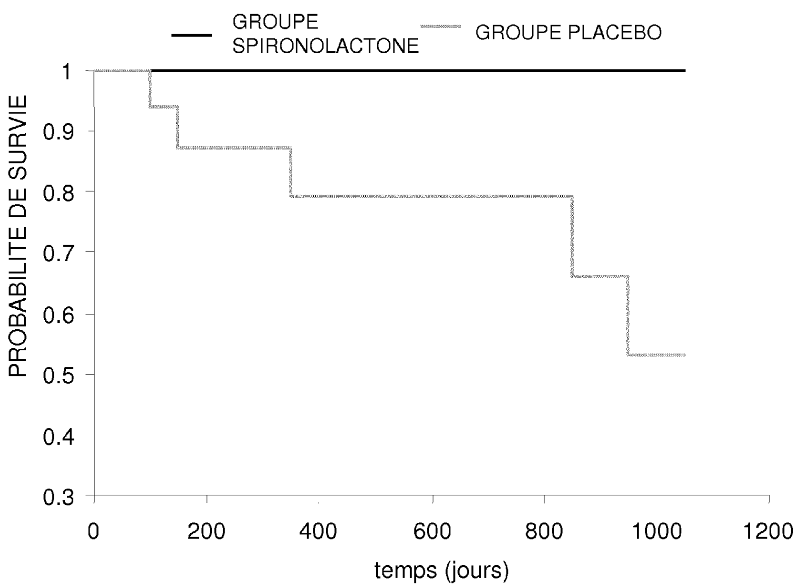


EP 2 567 701 A2

Printed by Jouve, 75001 PARIS (FR)

## Description

L'invention concerne de nouvelles compositions comprenant un antagoniste de l'aldostérone selon une posologie particulière pour le traitement de l'insuffisance cardiaque des animaux mammifères non humains.

Les maladies cardiaques sont fréquentes chez les mammifères non humains, tels que le chien et le chat. Celles-ci peuvent engendrer une insuffisance cardiaque. L'insuffisance cardiaque correspond à un syndrome dans lequel une anomalie de la fonction cardiaque entraine à terme une incapacité du coeur à assurer un débit sanguin suffisant pour couvrir les besoins énergétiques de l'organisme. Cette défaillance peut être le reflet d'une anomalie de la contraction du muscle cardiaque ventriculaire (dysfonction systolique) ou de remplissage du coeur (dysfonction diastolique), voire des deux mécanismes.

En médecine vétérinaire, la gravité de l'insuffisance cardiaque est estimée sur le plan fonctionnel selon, entre autres, la classification ISACHC (International Small Animal Cardiac Health Council) en trois classes. La classe I, dite asymptomatique, est seulement détectable du fait de la présence de signes de cardiopathie décelables à l'examen, tels que par exemple un souffle cardiaque ou une cardiomégalie. La classe II correspond à une insuffisance cardiaque débutante ou modérée; Elle est détectée par l'apparition de symptômes congestifs après effort. La classe III correspond à une insuffisance cardiaque avancée ou sévère et se traduit par l'apparition de symptômes cliniques même au repos, avec la présence d'ascite et d'oedème pulmonaire.

Au début de l'évolution de la maladie, la fonction cardiaque est maintenue par des mécanismes compensateurs, dont un des principaux est le système rénine-angiotensine-aldostérone (SRAA), via son rôle primordial dans le maintien de la volémie. Le SRAA est en fait une régulation endocrinienne cardiorénale qui maintient l'homéostasie hydrosodée de l'organisme, c'est-à-dire l'équilibre entre les électrolytes (ions sodium ($Na^+$), ion potassium ($K^+$), ion magnésium ($Mg^{2+}$)) et l'eau. Il fonctionne par une cascade de régulation endocrinienne et enzymatique. L'activation du SRAA débute par la sécrétion d'une enzyme par le rein, la rénine, lors de la baisse de la pression dans l'artère rénale. Cependant, il existe aussi d'autres stimuli, tels que la baisse de la natrémie au niveau du tube contourné distal ou la stimulation des cellules juxta-glomérulaires par le système béta-adrénergique. La rénine clive l'angiotensinogène qui est sécrété par le foie, pour donner un décapeptide inactif appelé angiotensine I. L'angiotensine I est ensuite transformée en angiotensine II principalement au niveau du poumon par l'enzyme de conversion de l'angiotensine (ECA). L'angiotensine II agit en se fixant sur ses récepteurs transmembranaires et favorise l'élévation de la pression artérielle par différents mécanismes. L'angiotensine II a notamment un puissant effet vasoconstricteur des artérioles, elle stimule la sécrétion d'aldostérone, une hormone sécrétée par les glandes surrénales qui provoque une augmentation de la volémie par réabsorption de sodium et d'eau au niveau des reins, dans le tube contourné distal et le tube collecteur, elle stimule également la sécrétion de vasopressine, une hormone antidiurétique, qui limite la perte d'eau dans les urines, et enfin inhibe en retour la sécrétion de la rénine.

Lors de l'insuffisance cardiaque, la synthèse d'aldostérone est augmentée suite à l'activation du système rénine-angiotensine. L'aldostérone, une hormone minéralocorticoïde synthétisée par les glandes surrénales, le coeur, les vaisseaux sanguins et le cerveau, agit en se fixant sur des récepteurs minéralcorticoïdes. Les principaux effets biologiques de l'aldostérone sont :

- sur le rein, la stimulation de la réabsorption de sodium et d'eau et de l'excrétion de potassium et de magnésium. La conséquence est une augmentation de la volémie.
- sur le coeur et sur les vaisseaux, une action directe qui conduit au remodelage tissulaire du myocarde et de l'endothélium vasculaire ainsi qu'au développement de fibrose dans le myocarde. Ces effets dépendent de la fixation de l'aldostérone sur les récepteurs minéralcorticoïdes.

En médecine humaine, les thérapies standards de l'insuffisance cardiaque utilisées sont entre autres les inhibiteurs de l'enzyme de conversion de l'angiotensine (IEC), les béta bloquants, les diurétiques, les vasodilatateurs, les inotropes, les digitaliques, et les hypertenseurs. Les inhibiteurs de l'Enzyme de Conversion de l'Angiotensine (IEC) tels que le captopril, l'enalapril, le bénazepril, le lisinopril, ou le ramipril, permettent de réguler la cascade du SRAA et ainsi la pression artérielle. De nombreux essais cliniques à grande échelle ont permis de démontrer l'efficacité des IEC: ils permettent d'augmenter significativement le taux de survie lors d'insuffisance cardiaque. Par contre, ils présentent des contre-indications telles que notamment l'hyperkaliémie.

Le traitement standard de l'insuffisance cardiaque humaine consiste en la combinaison d'un IEC et d'un diurétique. De nombreux diurétiques sont actuellement disponibles: les diurétiques de l'anse tels que le furosémide, le torsémide, le bumétanide, les diurétiques thiazidiques, tels que l'hydrochlorothazide ou le chlortalidone, ou encore les épargnants du potassium, tels que le triamterène, l'amiloride, l'éplérenone, et la spironolactone.

Les effets d'un antagoniste de l'aldostérone, la spironolactone, ont été également évalués lors de l'insuffisance cardiaque. Des études expérimentales ont démontré précédemment les effets délétères de l'aldostérone sur le rein et l'appareil cardiovasculaire. Des essais ont donc été réalisés en s'appuyant sur l'hypothèse que le blocage des effets

de l'aldostérone pourrait avoir des effets bénéfiques lors de l'insuffisance cardiaque, et en particulier permettre une amélioration de la fonction cardiaque et une diminution de l'incidence des troubles du rythme.

Il a été montré chez l'homme, que lorsque la spironolactone est administrée à des doses ayant un effet diurétique (≥ 50 mg), seule ou en combinaison avec un IEC, elle présente des effets secondaires d'hyperkaliémie incompatibles avec le traitement de l'insuffisance cardiaque et notamment les IEC. L'étude clinique multicentrique RALES (Randomized Aldactone Evaluation Study) a démontré le bénéfice clinique de la spironolactone utilisée à des doses faibles dites sub-diurétiques chez des patients humains atteints d'insuffisance cardiaque. Plus précisément, l'étude RALES décrit l'utilisation de la spironolactone à doses sub-diurétiques de 1 à 25mg/jour et d'un IEC pour le traitement de l'insuffisance cardiaque humaine. De telles combinaisons sont également décrites dans la demande internationale WO 96/24373 et le brevet EP 808172B1. Dans le cadre de l'étude RALES, 1663 patients atteints d'insuffisance cardiaque sévère, ayant une fraction d'éjection ventriculaire gauche inférieure à 35%, traités avec un IEC, des diurétiques de l'anse, et parfois avec de la digoxine, ont été inclus dans une étude en double aveugle contre placebo. 822 ont reçu 25 mg de spironolactone et 841 un placebo. L'étude rapporte 386 morts dans le groupe placebo (46%), contre 284 dans le groupe spironolactone (35%). L'analyse de survie montre que le risque de mortalité est réduit de 30% chez les patients recevant de la spironolactone par rapport à un groupe placebo.

S'il était connu que l'administration de spironolactone entraîne un risque accru d'hyperkaliémie chez les sujets humains atteints d'insuffisance cardiaque, il est maintenant établi que seules de faibles doses quotidiennes de spironolactone peuvent être utilisées pour ces traitements. Elles constituent en quelque sorte un compromis pour réduire l'effet de l'aldostérone sur la physiopathologie de l'insuffisance cardiaque tout en évitant les effets secondaires, notamment d'hyperkaliémie, dus à des dosages élevés de l'antagoniste de l'aldostérone. Il a donc été établi que, chez les patients humains, les doses thérapeutiques efficaces de spironolactone suffisantes pour observer un effet protecteur, mais assez faibles au regard des doses diurétiques, doivent être en général de 12,5 à 50 mg par jour et par patient humain. Toutefois et contrairement à ce qui avait été établi préalablement en terme de dosage des antagonistes de l'aldostérone pour le traitement de l'insuffisance cardiaque humaine, il a été découvert qu'il était possible, pour un sous-groupe de patients ou sujets particuliers, constitués d'animaux mammifères non humains, de réduire de manière très importante les risques de mortalité et/ou de morbidité, en utilisant des doses d'antagonistes d'aldostérone supérieures aux doses préalablement utilisées, et ceci sans toutefois induire de variation significative de la kaliémie ou avec de faibles variations de kaliémie chez ces sujets. En effet, de manière surprenante, aucun effet secondaire d'hyperkaliémie n'a été observé chez les animaux mammifères non humains ayant reçu des doses importantes d'antagonistes de l'aldostérone. En revanche, une efficacité supérieure inattendue en termes de survie a été démontrée avec une réduction significative des risques de mortalité et morbidité.

## RESUME DE L'INVENTION

La présente invention a donc pour objet une nouvelle composition vétérinaire comprenant un antagoniste de l'aldostérone administré selon une posologie prescrite, et un véhicule pharmaceutiquement acceptable, destinée au traitement de sujets mammifères non humains atteints d'insuffisance cardiaque.

Plus précisément, la posologie ou dose thérapeutique efficace d'antagoniste de l'aldostérone est supérieure à 1mg/kg/jour et inférieure à 5 mg/kg/jour, comprise entre 1,5 et 5mg/kg/jour, 1,8 et 5mg/kg/jour, 1,5 et 4 mg/kg/jour, 1,5 et 3 mg/kg/jour, de préférence comprise entre 2 et 5 mg/kg/jour, et de manière encore plus préférentielle d'environ 2 mg/kg/jour ou 4mg/kg/jour en une seule prise. Cette dose thérapeutique peut être associée à une dose thérapeutique efficace d'une thérapie standard de l'insuffisance cardiaque, telle que des IEC, des antagonistes du récepteur AT-1 de l'angiotensine II (ARA-II ou sartans), des digitaliques, des inotropes, des inodilatateurs, des diurétiques, des vasodilatateurs, des béta bloquants et/ou des antagonistes calciques.

Les compositions selon la présente invention sont particulièrement utiles pour le traitement et/ou la prévention de l'insuffisance cardiaque chez les animaux mammifères non humains, tels que les chiens, les chats et les chevaux, et de manière générale tous les animaux de compagnie. Elles permettent notamment de réduire le risque de mortalité, et/ou de morbidité, sans provoquer d'effets secondaires d'hyperkaliémie. Il a été déduit que le risque de mortalité est réduit de 50 à 80%, de 55 à 80%, de 60 à 80%, de 65 à 80%, de 70 à 80%, ou de 75 à 80% chez les chiens recevant de la spironolactone, ses dérivés, ou ses métabolites, à la posologie prescrite selon l'invention par rapport à un groupe placebo.

La présente invention a également pour objet des kits à usage vétérinaire destinés au traitement de sujets mammifères non humains atteints d'insuffisance cardiaque, ayant au moins un compartiment pour un conditionnement séparé ou non, des doses quotidiennes d'antagoniste de l'aldostérone seul ou en association une thérapie standard de l'insuffisance cardiaque, telle que des IEC, des antagonistes du récepteur AT-1 de l'angiotensine II, des digitaliques, des inotropes, des inodilatateurs, des diurétiques, des vasodilatateurs, des béta bloquants et/ou des antagonistes calciques. Le compartiment peut ainsi comprendre une dose quotidienne d'antagoniste de l'aldostérone supérieure à 1mg/kg/jour et inférieure à 5 mg/kg/jour, comprise entre 1,5 et 5mg/kg/jour, 1,8 et 5mg/kg/jour, 1,5 et 4 mg/kg/jour, 1,5

et 3 mg/kg/jour, de préférence comprise entre 2 et 5 mg/kg/jour, et de manière encore plus préférentielle d'environ 2 mg/kg/jour ou 4mg/kg/jour en une seule prise.

De plus, la présente invention a pour objet l'utilisation de quantités thérapeutiques efficaces d'un antagoniste de l'aldostérone seul ou en association avec des IEC, des antagonistes du récepteur AT-1 de l'angiotensine II, des digitaliques, des inotropes, des inodilatateurs, des diurétiques, des vasodilatateurs, des béta bloquants et/ou des antagonistes calciques, en vue de la préparation d'un médicament vétérinaire destiné à traiter et/ou prévenir l'insuffisance cardiaque, et/ou à réduire les taux de mortalité et/ou de morbidité des sujets animaux mammifères non humains atteints d'insuffisance cardiaque, sans provoquer d'effets secondaires d'hyperkaliémie. La dose thérapeutique efficace d'antagoniste de l'aldostérone est supérieure à 1mg/kg/jour et inférieure à 5 mg/kg/jour, comprise entre 1,5 et 5mg/kg/jour, 1,8 et 5mg/kg/jour, 1,5 et 4 mg/kg/jour, 1,5 et 3 mg/kg/jour, de préférence comprise entre 2 et 5 mg/kg/jour, et de manière encore plus préférentielle d'environ 2 mg/kg/jour ou 4mg/kg/jour en une seule prise.

Un autre objet de la présente invention consiste en une méthode de traitement des stades débutants d'insuffisance cardiaque chez les mammifères non humains comprenant l'administration de doses thérapeutiques efficaces d'un antagoniste de l'aldostérone supérieures à 1mg/kg/jour et inférieures à 5 mg/kg/jour, comprises entre 1,5 et 5mg/kg/jour, 1,8 et 5mg/kg/jour, 1,5 et 4 mg/kg/jour, 1,5 et 3 mg/kg/jour, de préférence comprises entre 2 et 5 mg/kg/jour, et de manière encore plus préférentielle d'environ 2 mg/kg/jour ou 4mg/kg/jour en une seule prise.

Enfin, un dernier objet de la présente invention consiste en une méthode de réduction des taux de mortalité et/ou de morbidité des sujets animaux mammifères non humains atteints d''insuffisance cardiaque comprenant l'administration de doses thérapeutiques efficaces d'un antagoniste de l'aldostérone seul ou en association avec des IEC, des antagonistes du récepteur AT-1 de l'angiotensine II, des digitaliques, des inotropes, des inodilatateurs, des diurétiques, des vasodilatateurs, des béta bloquants et/ou des antagonistes calciques. L'antagoniste de l'aldostérone est administré en une dose quotidienne supérieure à 1mg/kg/jour et inférieure à 5 mg/kg/jour, comprise entre 1,5 et 5mg/kg/jour, 1,8 et 5mg/kg/jour, 1,5 et 4 mg/kg/jour, 1,5 et 3 mg/kg/jour, de préférence comprise entre 2 et 5 mg/kg/jour, et de manière encore plus préférentielle d'environ 2 mg/kg/jour ou 4mg/kg/jour en une seule prise par jour. Selon cet objet, le risque de mortalité est réduit d'un pourcentage d'au moins 50%. De manière plus préférentielle, le pourcentage de réduction du risque de mortalité est compris entre environ 80% et 50%, 80% et 55%, 80 et 60%, 80% et 65%, 80% et 70%, ou entre 80% et 75%, et est par exemple d'environ 80%, 73%, 67%, 65 %, ou 59%. Selon cet objet, le risque de morbi-mortalité est réduit d'au moins 40% ou d'au moins 46%.

## BREVE DESCRIPTION DES FIGURES

**Figure 1:** Graphe semi-logarithmique des concentrations plasmatiques de spironolactone sur 48 heures chez le rat, le chien et le singe après administration de 2mg/kg/jour de 22-$^{14}$Cspironolactone marquée par voie orale ou intraveineuse (● - ● : matériel total $^{14}$C ; ▲- ▲: matériel $^{14}$C éthyl acétate; ■ - ■: canrénone).

**Figure 2 :** Graphe semi-logarithmique de la concentration de canrénone par rapport au temps après administration de doses de spironolactone de 0,8mg/kg, 2mg/kg, et 8mg/kg.

**Figure 3 :** Graphe représentant la relation dose réponse entre la dose de spironolactone et le ratio log ($[Na^+]_{urinary}$ x 10/$[K^+]_{urinary}$) chez 15 chiens en hyperaldostéronémie après une seule administration de spironolactone par jour. Le graphe montre que la spironolactone permet de restaurer le ratio Log (Nax10/K) dans un intervalle de 0 à 6 heures.

**Figure 4 :** Graphe représentant les probabilités de survie obtenues sur une durée de 14-15 mois, dans le cadre d'études cliniques conduites sur un groupe de chiens ayant reçu une composition orale de spironolactone (2mg/kg/jour) et un IEC (Groupe spironolactone) et un second groupe ayant reçu un placébo et un IEC (p=0,011) ; La spironolactone réduit de 65% le risque de mortalité.

**Figure 5 :** Graphe représentant les taux de mortalité à 14-15 mois, dans le cadre d'études cliniques conduites sur un groupe de chiens ayant reçu une composition orale de spironolactone (2mg/kg/jour) et un IEC (Groupe spironolactone) et un second groupe ayant reçu un placébo et un IEC (p=0,0029).

**Figure 6 :** Graphe représentant les probabilités de survie obtenues sur une durée de 3 ans, dans le cadre d'études cliniques conduites sur un groupe de chiens ayant reçu une composition orale de spironolactone (2mg/kg/jour) et un IEC (Groupe spironolactone) et un second groupe ayant reçu un placébo et un IEC (p=0,017) ; La spironolactone réduit de 59% le risque de mortalité.

**Figure 7 :** Graphe représentant les probabilités de survie obtenues sur une durée de 3,5 ans, dans le cadre d'études cliniques conduites sur un groupe de chiens traités dès le stade I de l'insuffisance cardiaque et ayant reçu une composition orale de Spironolactone (2mg/kg/jour) et un IEC (Groupe spironolactone) et un second groupe ayant reçu un placébo et un IEC.

**Figure 8 :** Graphe représentant les taux de morbidité-mortalité obtenus à 14-15 mois, dans le cadre d'études cliniques conduites sur un groupe de chiens ayant reçu une composition orale de spironolactone (2mg/kg/jour) et un IEC (Groupe spironolactone) et un second groupe ayant reçu un placébo et un IEC. Le taux de morbi-mortalité est obtenu en additionnant le nombre de chiens morts, euthanasiés ou sortis de l'essai pour aggravation de leur insuffisance cardiaque.

**Figure 9:** Graphe représentant la progression de la concentration de potassium plasmatique moyenne ou kaliémie observée dans les différents groupes de chiens traités avec une composition orale de spironolactone (2mg/kg/jour) et un IEC (Groupe spironolactone) en comparaison avec les groupes de chiens qui ont reçu un placébo et un IEC ($p<0,05$).

**DESCRIPTION DETAILLEE**

La présente invention a pour objet une nouvelle composition vétérinaire destinée au traitement de sujets mammifères non humains atteints d'insuffisance cardiaque comprenant un antagoniste de l'aldostérone et un véhicule pharmaceutiquement acceptable, dans laquelle la proportion thérapeutique efficace d'antagoniste de l'aldostérone est supérieure à 1mg/kg/jour et inférieure à 5 mg/kg/jour, comprise entre 1,5 et 5mg/kg/jour, 1,8 et 5mg/kg/jour, 1,5 et 4 mg/kg/jour, 1,5 et 3 mg/kg/jour, de préférence comprise entre 2 et 5 mg/kg/jour, et de manière encore plus préférentielle d'environ 2 mg/kg/jour ou 4mg/kg/jour en une seule prise.

La présente invention a également pour objet de nouvelles compositions vétérinaires destinées au traitement de sujets mammifères non humains atteints d'insuffisance cardiaque comprenant un antagoniste de l'aldostérone en association avec une thérapie standard pour le traitement de l'insuffisance cardiaque, telle que par exemple des IEC, des antagonistes du récepteur AT-1 de l'angiotensine II (ARA-II ou sartans), des digitaliques, des inotropes, des inodilatateurs, des diurétiques, des vasodilatateurs, des béta bloquants et/ou des antagonistes calciques, et un véhicule pharmaceutiquement acceptable.

Selon la présente invention, les antagonistes de l'aldostérone incluent tous les agents qui sont susceptibles de se fixer sur le récepteur de l'aldostérone (appelé aussi antagoniste du récepteur de l'aldostérone) et agir ainsi en tant qu'antagoniste compétitif de l'aldostérone par fixation sur le site de liaison des minéralocorticoïdes. A titre d'exemples d'inhibiteurs d'aldostérone, on peut citer les composés de type spironolactone qui comprennent un cycle lactone lié à un noyau stéroïde.

Les nouvelles compositions vétérinaires sont donc destinées au traitement de sujets mammifères non humains atteints d'insuffisance cardiaque comprenant un antagoniste du récepteur de l'aldostérone, un inhibiteur d'enzyme de conversion de l'angiotensine (IEC), et un véhicule pharmaceutiquement acceptable. Les compositions en question comprennent une dose thérapeutique efficace d'antagoniste du récepteur de l'aldostérone d'environ 0,88 à 5 mg/kg/jour, et de préférence d'environ 2 mg/kg/jour. Celles-ci comprennent par ailleurs une dose thérapeutique efficace d'IEC d'environ 0,1 à 0,6 mg/kg/jour, et de préférence d'environ 0,25 mg/kg/jour. Son l'invention on utilise des quantités thérapeutiquement efficaces d'un antagoniste du récepteur de l'aldostérone et d'un inhibiteur d'enzyme de conversion de l'angiotensine, en vue de la préparation d'un médicament vétérinaire destiné à réduire les taux de mortalité et/ou de morbidité des sujets animaux mammifères non humains atteints d'insuffisance cardiaque, caractérisée en ce que la dose thérapeutique efficace d'antagoniste du récepteur de l'aldostérone est comprise entre environ 0,88 et 5 mg/kg/jour, et de préférence d'environ 2 mg/kg/jour. La méthode de réduction des taux de mortalité et/ou de morbidité des sujets animaux mammifères non humains atteints d''insuffisance cardiaque comprend l'administration de doses thérapeutiques efficaces d'un antagoniste du récepteur de l'aldostérone et d'un inhibiteur d'enzyme de conversion de l'angiotensine, l'antagoniste du récepteur de l'aldostérone étant administré en une dose quotidienne comprise entre environ 0,88 et 5 mg/kg/jour, et de préférence d'environ 2 mg/kg/jour.

L'efficacité thérapeutique de la présente invention peut être exprimée en taux de mortalité observés mais aussi en risque de mortalité. Ainsi, dans la présente invention le risque de mortalité est réduit d'un pourcentage d'au moins 50% et le taux de mortalité d'au plus 34%. De manière plus préférentielle, le pourcentage de réduction du risque de mortalité est compris entre environ 80% et 50%, 80% et 55%, 80 et 60%, 80% et 65%, 80% et 70%, ou entre 80% et 75%, et est par exemple d'environ 80%, 73%, 67%, 65 %, ou 59%. De la même manière, les taux de mortalité plus préférentiels obtenus sont compris entre environ 34% et 0%, et sont par exemple d'environ 34%, 30%, 20%, 15 %, 9%, 6% ou 0%. Selon cet objet, le risque de morbi-mortalité est réduit d'au moins 40% ou d'au moins 46% à 50%.

Les antagonistes ou inhibiteurs compétitifs de l'aldostérone (susceptibles de se fixer de manière compétitive

sur le récepteur de l'aldostérone comme cela est décrit ci-dessus) peuvent répondre à

(i) formule générale I suivante :

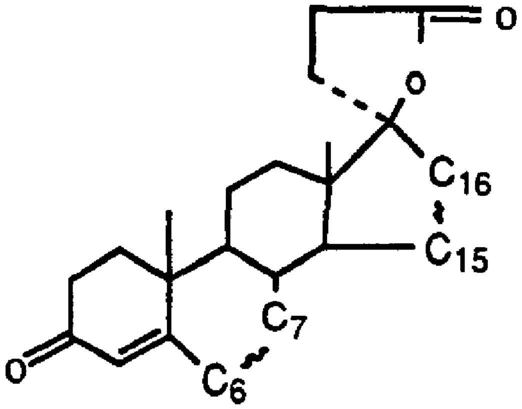

(I)

dans laquelle

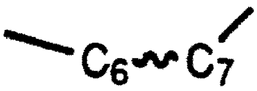 est 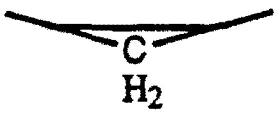 or 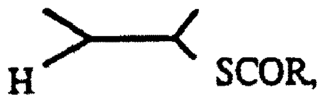

dans laquelle R représente un alkyl inférieur de 1 à 5 atomes de carbone, et dans laquelle

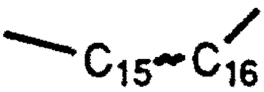 est 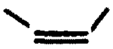 , 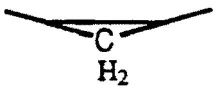 or 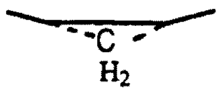 .

Les résidus alkyls inférieurs peuvent être linéaires ou non, de préférence des groupes méthyl, un éthyl, et un n-propyl. Des exemples de composés de type spironolactone appartenant à la formule I sont listés ci-dessous. Les méthodes de production de ces composés sont bien connus dans le domaine et sont entre autres décrites dans le brevet US 4,129,564.

- le 7α-Acétylthio-3-oxo-4,15-androstadiène-[17(ß-1')-spiro-5']perhydrofuran-2'-one;
- le 3-Oxo-7α-propionylthio-4,15-androstadiène-[17((ß-1')-spiro 5'] perhydrofuran -2'-one;
- le 6ß,7ß-Méthylène-3-oxo4,15-androstadiène-[17((ß-1')-spiro-5'] perhydrofuran-2'-one;
- le 15α,16α-Méthylène-3-oxo-4,7α-propionylthio-4androstene[17(ß-1')-spiro-5']perhydrofuran-2'-one;
- le 6ß,7ß,15α,16α-Diéthylène-3-oxo-4-androstene [17(ß-1')-spiro-5']perhydrofuran-2'-one;
- le 7α-Acétylthio-15ß,16ß-Méthylène-3-oxo-4-androstene-[17(ß-1')-spiro-5']perhydrofuran-2'-one;
- le 15ß,16ß-Methyléne-3-oxo-7ss-propionylthio-4-androstene-[17(ß-1')-spiro-5']perhydrofuran-2'-one; et
- le 6ß,7ß,15ß,16ß-Diméthylène-3-oxo-4-androstène-[17(ß1')-spiro-5']perhydrofuran-2'-one.
- 10,13-diméthylspiro[2,8,9,11,12,14,15,16-octahydro-1 H-cyclopenta[a]phenanthrene-17,5'-oxolane]-2',3-dione (canrénone).

(ii) formule générale II suivante :

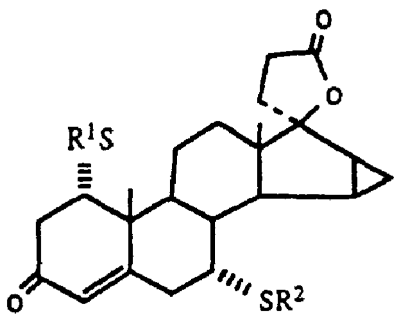

(II)

dans laquelle le radical R1 est un alkyl ou un acyl en C1-C3 et R2 est un hydrogène ou un alkyl en C1-C3. Des composés appartenant à cette famille sont par exemple le 1α-acétylthio-15ß,16ß-méthylène-7α - méthylthio-3-oxo-17α -pregn-4-ene-21,17-carbolactone; et le 15ß,16ß-Méthylène-1α,7α-diméthylthio-3-oxo-17α-pregn- 4-ène-21,17-carbolactone.

(iii) formule générale III suivante :

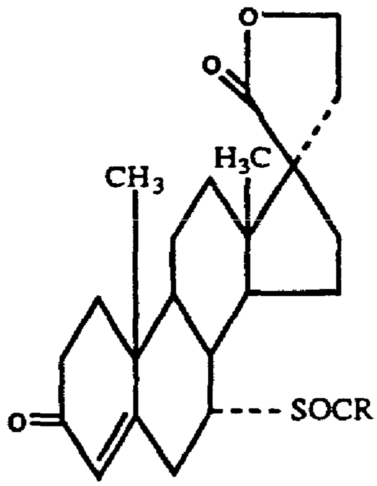

or

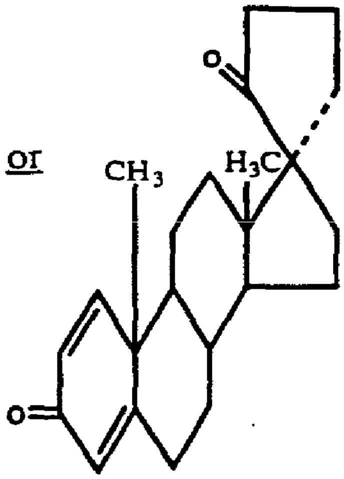

(III)

dans laquelle R est un alkyl inférieur, de préférence un groupement méthyle, éthyle, propyle, et butyle. A titre d'exemples, on peut citer :

- l'acide 3ß, 21-dihydroxy-17α-pregna-5,15-diene-17 carboxylique y-lactone;
- l'acide 3ß, 21-dihydroxy-17α-pregna-5,15-diene-17-carboxylique lactone 3-acetate;
- l'acide 3ß, 21-dihydroxy-17α-pregn-5-ene-17-carboxylique γ-lactone;
- l'acide 3ß, 21-dihydroxy-17α-pregn-5-ene-17-carboxylique γ-lactone 3-acetate;
- l'acide 21-hydroxy-3-oxo-17α -pregn-4-ene-17-carboxylique γ-lactone;
- l'acide 21-hydroxy-3-oxo-17α -pregna-4, 6-diene-17-carboxylique γ-lactone;
- l'acide 21-hydroxy-3-oxo-17α-pregna-1,4-diene-17 carboxylique γ-lactone;
- l'acide 7α-acylthio-21-hydroxy-3-oxo-17α-pregn-4-ene-17carboxylique γ-lactones; et
- l'acide 7α-acétylthio-21-hydroxy-3-oxo-17a-pregn-4-ene-17-carboxylique γ-lactone.

(iv) formule générale IV suivante :

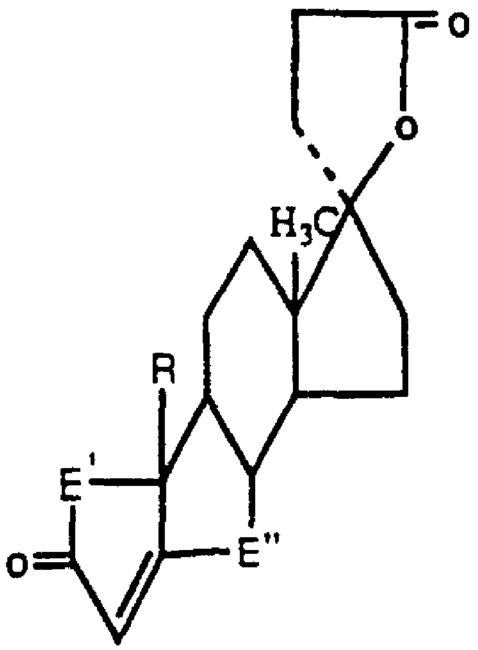

(IV)

dans laquelle E' est un groupement choisi parmi l'éthylène, le vinylène, et des radicaux thioethylène alkanoyls inférieurs ;

dans laquelle E" est un groupement choisi parmi l'éthylène, le vinylène, et des radicaux thioethylène alkanoyls inférieurs, et R est un radical méthyl, sauf lorsque E' et E" sont des radicaux éthylènes.

(v) formule générale V suivante, parmi lesquels on peut citer par exemple, le 1-acétylthio-17α-(2-carboxyethyl)-17ß-hydroxy-androst-4-en-3-one lactone.

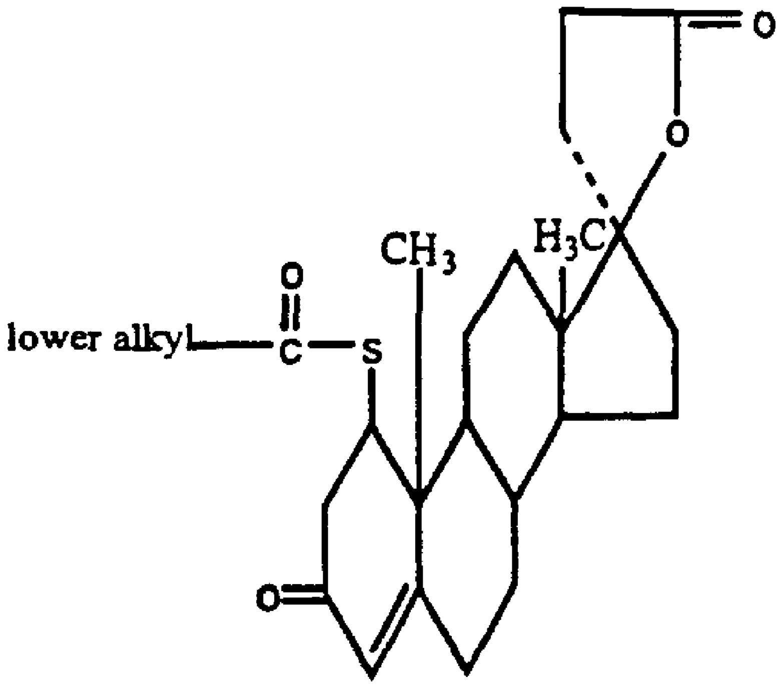

(V)

(vi) formule générale VI suivante. A titre d'exemples, on peut citer le 7α-acétylthio-17a-(2-carboxyéthyl)-17ß-hydroxy-androst-4-en-3-one lactone; 7α-acétylthio-17α-(2-carboxyéthyl)-17ß-hydroxy-androst-4-en-3-one lactone; 1α,7α-diacétylthio-17α-(2-carboxyéthyl)-17ss-hydroxy-androsta-4,6-dien-3-one lactone; 7α-acétylthio-17α-(2-carboxyethyl)-17ß-hydroxy- androsta-1,4-dien-3-one lactone; 7α-acétylthio-17α-(2-carboxyéthyl)-17ß-hydroxy-19-norandrost-4-en-3-one lactone; et 7α-acétylthio-17α-(2-carboxyéthyl)-17ß-hydroxy-6α-méthylandrost-4-en-3-one lactone.

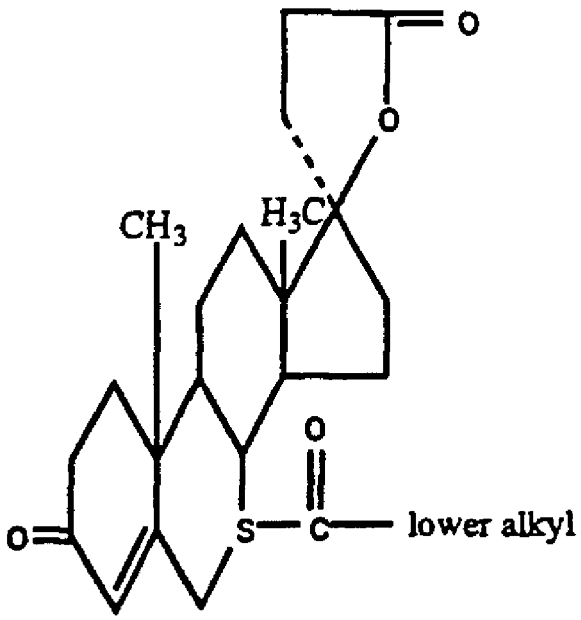

(VI)

(vii) formule générale VII suivante :

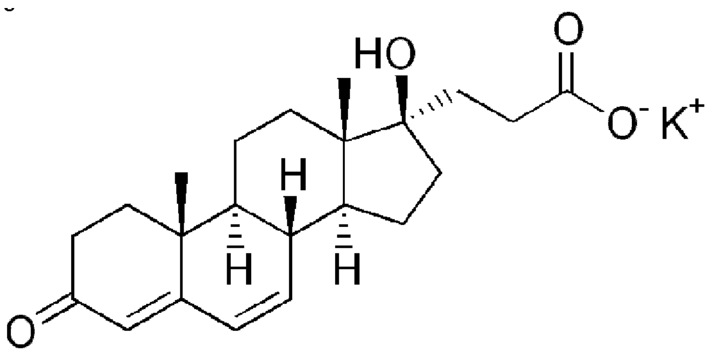

(VII)

A titre d'exemple, on peut citer le potassium 3-[(8R,9S,10R,13S,14S,17R)-17-hydroxy-10,13-dimethyl-3-oxo-2,8,9,11,12,14,15,16-octahydro-1H-cyclopenta[α] phenanthren-17-yl] propanoate (caneroate). Les compositions vétérinaires selon la présente invention comprennent de préférence la spironolactone, ou l'éplérénone en tant qu'antagoniste de l'aldostérone. Tout au long de la présente description, les termes spironolactone et éplérénone englobent également les dérivés ou métabolites de ces composés. De préférence, la spironolactone utilisée selon la présente invention est un composé stéroïde synthétique 17-lactone appartenant à la famille (I) décrite ci-dessus. De manière encore plus préférentielle, on utilise la 7α-acétylthio-3-oxo-17α-pregn-4-ène-21,17-carbolactone. La spironolactone est bien connue en médecine humaine, et elle est commercialisée sous les dénominations commerciales Aldactone®, Novo-Spiroton®, Spiractin®, Spirotone®, et Berlactone®. La formule générale chimique est comme suit (http://www.chemblink.com):

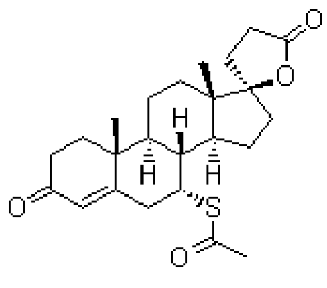

L'éplérénone, également désignée par époxymexrénone, est un dérivé époxy: 9, 11-epoxy- spirolactone (US 4,559,332). Le nom chimique complet est pregn-4-ene-7,21-dicarboxylic acid, 9,11-epoxy-17-hydroxy-3-oxo, γ-lactone,

méthyl ester (7α, 11α, 17α). Elle est commercialisée, en médecine humaine, entre autre sous la dénomination commerciale Inspra®. La formule générale est la suivante (http://www.chemblink.com):

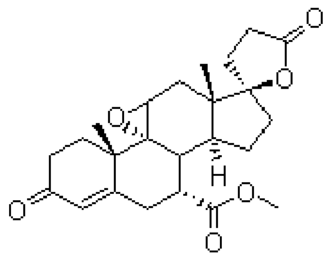

Selon l'invention, on entend par spironolactone, le composé en tant que tel, ses dérivés et/ou ses métabolites. A titre d'exemples de dérivés des spironolactone, on peut citer les isomères optiquement actifs de la spironolactone, les dérivés mono ou bis-cyclopropyl de la spironolactone, ou les dérivés époxy, tels que le 9α,11α-époxy spironolactone ou éplérénone, ou plus généralement tous les dérivés fonctionnalisés de la spironolactone actifs, c'est-à-dire qui présentent l'activité thérapeutique de la spironolactone selon la présente invention. A titre d'exemples des métabolites de la spironolactone, on peut citer sans limitation, la canrénone, l'acide canrénoique, la 15β-OH canrénone, la 21-OH canrénone, le canrénoate de potassium, la 7α-thio-spironolactone, la 7α-thiométhylspironolactone, ou la 6-β-hydroxy-7-α-thiométhyl spironolactone.

Les composés de type spironolactone, tels que la spironolactone, l'éplérénone, les dérivés, ou métabolites de ces composés sont administrés à des doses supérieures à celles qui sont conventionnellement utilisées pour les traitements de l'insuffisance cardiaque chez l'homme. Ces doses sont généralement qualifiées de doses diurétiques et induisent chez le patient humain des effets secondaires d'hyperkaliémie. Il a été découvert selon la présente invention que les doses des composés de type spironolactone ayant un effet diurétique chez les animaux mammifères non humains qui sont identiques à celles provoquant un effet diurétique chez l'homme n'induisent pas, contrairement à ce qui est observé chez l'homme, d'effets secondaires d'hyperkaliémie dans le sous groupe de patients constitués des animaux mammifères non humains.

Les compositions selon la présente invention qui comprennent de préférence la spironolactone ou l'éplérénone ou les dérivés, les métabolites de ces composés en tant qu'antagoniste de l'aldostérone sont particulièrement utiles lorsqu'elles sont administrées selon les posologies prescrites pour le traitement et/ou la prévention de l'insuffisance cardiaque chez les animaux non humains tels que le chien et/ou le chat, notamment dans les stades débutants de la pathologie. De préférence, les compositions selon la présente invention sont administrées avant l'apparition d'oedèmes chez ces animaux malades.

Chez l'homme les doses de spironolactone conventionnellement utilisées sont d'environ 1 à 25 mg/jour avec une moyenne d'administration quotidienne à 12,5 mg/jour, mais en aucun cas au delà de 50 mg/jour afin de ne pas provoquer d'hyperkaliémie chez l'homme. Les doses humaines sont parfois rapportées aux animaux par extrapolation allométrique, méthode qui prend en compte différents paramètres physiologiques tels que notamment la pharmacocinétique. L'équation allométrique couramment appliquée est comme suit :

$$\text{Log(Cl)} = 0{,}5408 \text{ x Log (BW)} - 0{,}2764$$

BW: Body Weight: poids

Par conséquent, la clairance (Cl) ou coefficient d'épuration plasmatique d'une substance, c'est-à-dire la capacité d'un organe à éliminer totalement une substance donnée d'un volume donné de plasma artériel par unité de temps est déduite de l'équation : $Cl = 0{,}5291x\ BW^{0{,}5408}$.

En utilisant cette équation allométrique, les clairances pour les chiens et les hommes sont calculées, avec un poids (BW) de 70kgs et de 10kgs respectivement pour l'homme et le chien. Les clairances ainsi calculées sont données dans le Tableau 1 ci-dessous.

Tableau 1 :

| Espèces | Poids (kg) | Clairance (L/h) |
|---|---|---|
| Chien | 10 | 1,831 |
| Humain | 70 | 5,253 |

Par conséquent, les doses de 12,5mg ; 25mg ; 50mg et 75mg (dose en mg/jour pour un patient humain) extrapolées à un chien (dose en mg/kg/jour), calculées selon l'équation suivante : $Dose_{chien} = (Dose_{humain} \times Cl_{chien}) / Cl_{humain}$, sont présentées dans le Tableau 2 ci-dessous :

Tableau 2

| Doses en mg/jour (patient humain) | Equivalent en mg/kg/jour (animaux mammifères non humains) | Effet thérapeutique chez l'homme |
|---|---|---|
| 12,5mg/jour | 0,436mg/kg/jour | Diminution significative de la morbidité/mortalité, sans hyperkaliémie |
| 25mg/jour | 0,871mg/kg/jour | Diminution significative de la morbidité/mortalité, sans hyperkaliémie |
| 50mg/jour | 1,743mg/kg/jour | Diminution significative de la morbidité/mortalité et apparition d'un effet diurétique (léger) et d'une hyperkaliémie (modérée) |
| 75mg/jour | 2,614mg/kg/jour | Diminution significative de la morbidité/mortalité et apparition d'un effet diurétique (modéré à important) et d'une hyperkaliémie (modérée à sévère) |

Selon la présente invention, les proportions thérapeutiques efficaces d'antagoniste de l'aldostérone, sans effets secondaires d'hyperkaliémie chez les animaux mammifères non humains, sont supérieures à 1mg/kg/jour et inférieures à 5 mg/kg/jour, comprises entre 1,5 et 5mg/kg/jour, 1,8 et 5mg/kg/jour, 1,5 et 4 mg/kg/jour, 1,5 et 3 mg/kg/jour, de préférence comprises entre 2 et 5 mg/kg/jour, et de manière encore plus préférentielle d'environ 2 mg/kg/jour ou 4mg/kg/jour en une seule prise.

En effet, les proportions thérapeutiquement efficaces sans effets secondaires transitoires chez les animaux mammifères non humains d'antagoniste du récepteur de l'aldostérone sont d'environ 0,88 à 5 mg/kg/jour, ou d'environ 1 à 5 mg/kg/jour, ou d'environ 1 à 4 mg/kg/jour, ou encore d'environ 1 à 3 mg/kg/jour et de préférence d'environ 2 mg/kg/jour ou d'environ 4 mg/kg/jour.

De telles doses de spironolactone sont généralement établies comme ayant également un effet diurétique chez l'homme, et ne peuvent donc pas être utilisées chez l'homme seules ou en combinaison avec des IEC, car elles provoquent un important effet secondaire d'hyperkaliémie chez les patients humains ainsi traités, qui n'est pas compatible avec le traitement souhaité de l'insuffisance cardiaque.

Il a été constaté de manière surprenante que de telles doses élevées de spironolactone supérieures à 1mg/kg/jour et inférieures à 5 mg/kg/jour, comprises entre 1,5 et 5 mg/kg/jour, 1,8 et 5 mg/kg/jour, 1,5 et 4 mg/kg/jour, 1,5 et 3 mg/kg/jour, de préférence comprises entre 2 et 5 mg/kg/jour, et de manière encore plus préférentielle d'environ 2 mg/kg/jour ou 4 mg/kg/jour et en administration toutes les 24 heures en une seule prise, n'induisent pas d'effets secondaires similaires d'hyperkaliémie chez les animaux mammifères non humains, ou n'induisent qu'une hyperkaliémie faible et transitoire. En effet, lorsque les animaux mammifères non humains sont traités avec les compositions selon la présente invention, les taux de potassium circulants restent substantiellement constants ou sont faiblement et transitoirement augmentés à des concentrations comprises entre 5,9 à 6,4 mmol/L ou bien comprises entre 6,5 et 7,5 mmol/L, et restent inférieurs à 7,5mmol/L. Aucun effet secondaire important d'hyperkaliémie n'a été observé lors du traitement des animaux mammifères non humains comme cela est démontré par ailleurs dans les exemples.

Les doses d'antagoniste de l'aldostérone, par exemple de spironolactone, d'éplérénone, des dérivés, ou métabolites de ces composés sont adaptées à chacun des mammifères traités en fonction du poids et de sorte à respecter la posologie prescrite par la présente invention supérieure à 1mg/kg/jour et inférieure à 5 mg/kg/jour, comprise entre 1,5 et 5 mg/kg/jour, 1,8 et 5 mg/kg/jour, 1,5 et 4 mg/kg/jour, 1,5 et 3 mg/kg/jour, de préférence comprise entre 2 et 5 mg/kg/jour, et de manière encore plus préférentielle d'environ 2 mg/kg/jour ou 4 mg/kg/jour en une seule prise.

Selon la présente invention, une seule administration par 24 heures d'antagoniste de l'aldostérone tel que la spironolactone à forte dose est effectuée, alors même que la concentration plasmatique de spironolactone diminue rapidement 4 heures après administration orale (Figure 1). Les antagonistes de l'aldostérone tels que la spironolactone, ses dérivés, ou ses métabolites, sont de préférence administrés aux animaux malades, en une seule prise une fois par

jour, par exemple lors de la prise d'aliments, soit mélangés à la ration alimentaire, soit directement dans la gueule après le repas. De préférence, les compositions selon la présente invention sont administrées aux stades précoces de l'insuffisance cardiaque et notamment avant l'apparition d'oedèmes chez ces animaux malades. Aucun effet secondaire d'hyperkaliémie n'a été observé chez les animaux ainsi traités.

Comme cela est démontré notamment dans l'Exemple 1 ci-après, la dose journalière optimale de spironolactone a été déterminée chez les animaux non humains tels que chez le chien par exemple. Cette dose est de manière surprenante bien supérieure à la dose couramment utilisée pour le traitement des patients humains. Cette dose optimale est supérieure à 1mg/kg/jour et inférieure à 5 mg/kg/jour, comprise entre 1,5 et 5 mg/kg/jour, 1,8 et 5 mg/kg/jour, 1,5 et 4 mg/kg/jour, 1,5 et 3 mg/kg/jour, de préférence comprise entre 2 et 5 mg/kg/jour, et de manière encore plus préférentielle d'environ 2 mg/kg/jour ou 4 mg/kg/jour, et peut aller jusqu'à 6 ou 7 mg/kg/jour pour le traitement des animaux non humains, avec un maximum à 8 mg/kg/jour.

L'utilisation de ces nouvelles posologies de spironolactone permet de rétablir le rapport sodium potassium plasmatique induit par hyperaldostéronémie reproduisant le modèle de l'insuffisance cardiaque. L'utilisation de ces nouvelles posologies de spironolactone permet de rétablir des concentrations urinaires de sodium et potassium physiologiques et d'obtenir une normalisation du rapport ($[Na^+]_{urinary}$ x 10/$[K^+]_{urinary}$). Les doses thérapeutiquement efficaces et non toxiques de spironolactone, ses dérivés, ou ses métabolites selon la présente invention sont supérieures à 1mg/kg/jour et inférieures à 5 mg/kg/jour, comprises entre 1,5 et 5 mg/kg/jour, 1,8 et 5 mg/kg/jour, 1,5 et 4 mg/kg/jour, 1,5 et 3 mg/kg/jour, de préférence comprises entre 2 et 5 mg/kg/jour, et de manière encore plus préférentielle d'environ 2 mg/kg/jour ou 4 mg/kg/jour et administrées toutes les 24 heures en une seule prise aux animaux afin de permettre la normalisation du rapport log ($[Na^+]_{urinary}$ x 10/$[K^+]_{urinary}$) et de traiter et/ou de prévenir les pathologies majeures des animaux non humains, que sont les insuffisances cardiaques.

Selon la présente invention, les compositions de spironolactone, ses dérivés, ou ses métabolites, administrées en une seule prise et à une dose supérieure à 1mg/kg/jour et inférieure à 5 mg/kg/jour, comprise entre 1,5 et 5 mg/kg/jour, 1,8 et 5 mg/kg/jour, 1,5 et 4 mg/kg/jour, 1,5 et 3 mg/kg/jour, de préférence comprise entre 2 et 5 mg/kg/jour, et de manière encore plus préférentielle d'environ 2 mg/kg/jour ou 4 mg/kg/jour sont particulièrement utiles pour traiter les animaux non humains atteints d'insuffisance cardiaque, sans effets secondaires d'hyperkaliémie. L'insuffisance cardiaque a pour origine des cardiopathies qui peuvent être de deux catégories : des cardiopathies congénitales ou des cardiopathies acquises. Les premières sont des malformations cardiaques congénitales. Contrairement aux affections congénitales, les cardiopathies acquises apparaissent au cours de la vie de l'animal, généralement tardivement (> 6-8 ans). Elles sont d'origines variées, mais deux affections prédominent très nettement chez le chien : la Maladie Valvulaire Dégénérative (MVD) et la cardiomyopathie Dilatée (CMD).

La MVD qui est aussi appelée endocardiose valvulaire, insuffisance valvulaire ou valvulopathie, représente 80% des cardiopathies des chiens. La MVD se caractérise par une altération des valvules atrioventriculaires (mitrale majoritairement, parfois mixte) entraînant un défaut d'étanchéité lors de la systole ventriculaire. Il y a alors une régurgitation de sang dans l'atrium qui entraîne une baisse du volume d'éjection systolique et une surcharge volumique dans l'atrium. Lorsque les lésions valvulaires progressent, on peut avoir également une atteinte et une rupture des cordages tendineux, qui aggravent la fuite valvulaire et le pronostic vital. Le premier moyen de détection de la MVD est l'auscultation; avec la fuite mitrale apparaît un souffle (systolique apexien gauche), dont l'intensité est corrélée à l'importance de la régurgitation, l'échocardiographie est aussi largement utilisée. Avec la progression de l'affection, on observe d'abord une dilatation atriale, puis une dilatation ventriculaire. A ce stade, la fonction systolique est altérée très précocement. Lorsque la valvule mitrale est touchée, l'insuffisance cardiaque résultante est d'abord gauche ; dans les cas avancés, elle peut devenir globale, gauche et droite. La mise en place des mécanismes compensateurs se fait progressivement, l'apparition d'une Insuffisance Cardiaque Congestive (ICC) est relativement tardive et induit des oedèmes et hypertension pulmonaires lors d'ICC gauche; des ascites lors d'insuffisance cardiaque droite ; l'évolution de la MVD se compte en mois ou en années.

La CMD est une affection primitive du myocarde des animaux non humains, des chiens ou des chats. Dans sa forme classique, elle se manifeste par un amincissement des parois du myocarde ventriculaire et une dilatation des cavités cardiaques. L'atteinte de la fonction systolique est précoce et profonde. La CMD est une affection d'évolution généralement très rapide, avec installation d'une ICC brutale et décompensée.

Les compositions de spironolactone, ses dérivés, ou ses métabolites telles que précédemment décrites permettent également de prévenir et/ou de traiter les pathologies du chat que sont les cardiomyopathies hypertrophiques félines (CMD, ou cardiomyopathies dilatées et/ou CMH, ou cardiomyopathies hypertrophiques). Ces dernières se caractérisent par un épaississement du myocarde ventriculaire qui réduit peu à peu le volume de la cavité ventriculaire. Le volume du sang que peut accepter la cavité est ainsi réduit, ce qui induit à terme, comme dans le cas de la CMD, à une insuffisance cardiaque congestive (ICC).

De préférence selon la présente invention, les compositions de spironolactone, ses dérivés ou ses métabolites, et dosages précédemment décrits sont particulièrement utiles pour traiter les animaux mammifères non humains atteints d'insuffisance cardiaque congestive à régurgitation valvulaire. Comme cela est décrit précédemment, les valvules cardiaques sont alors défaillantes et le coeur n'est plus capable d'assurer une perfusion suffisante des différents organes. Le sang stagne dans les veines, et du liquide plasmatique diffuse dans les tissus, provoquant oedèmes et épanchements.

Par animaux mammifères non humains, on entend de manière générale toutes les espèces de mammifères. De préférence, les compositions selon la présente invention sont destinées aux animaux de compagnie, tels que par exemple le chien, le chat, et le cheval.

Les compositions comprenant des doses élevées d'antagoniste de l'aldostérone, tel que la spironolactone, ses dérivés ou ses métabolites sont utilisées en association avec une thérapie standard pour le traitement de l'insuffisance cardiaque. De préférence, les compositions de spironolactone, ses dérivés, ou ses métabolites sont utilisées en association avec une thérapie standard pour le traitement de l'insuffisance cardiaque congestive.

Selon l'invention, on entend par thérapie standard de l'insuffisance cardiaque, des IEC, des antagonistes du récepteur AT-1 de l'angiotensine II (ARA-II ou sartans), des digitaliques, des inotropes, des inodilatateurs, des diurétiques, des vasodilatateurs, des béta bloquants et/ou des antagonistes calciques

Selon un mode de réalisation préféré, les compositions de spironolactone, ses dérivés, ou ses métabolites selon la présente invention sont utilisées en combinaison avec un IEC. Parmi les IEC, on peut citer notamment le Benazépril, l'Enalapril, le Captopril, le Cilazapril, le Fosinopril, l'Imidapril, le Lisinopril, le Moexipril, le Perindopril, le Quinapril, le Ramipril, le Spirapril, ou encore le Trandolapril. De préférence, le benazépril ou l'énalapril est utilisé dans les compositions selon la présente invention en combinaison avec la spironolactone, ses dérivés, ou ses métabolites. Les doses thérapeutiques efficaces d'inhibiteur d'enzyme de conversion de l'angiotensine utilisées dans les compositions sont d'environ 0,1 à 0,6 mg/kg/jour, de préférence d'environ 0,25 mg/kg/jour pour le benazépril et 0,5 mg/kg/jour pour l'énalapril.

Alternativement, les compositions de spironolactone, ses dérivés, ou ses métabolites selon la présente invention sont utilisées en combinaison avec des antagonistes du récepteur AT-1 de l'angiotensine II, également désignés ARA-II ou sartans. Ces composés agissent comme des inhibiteurs compétitifs de l'angiotensine II au niveau du récepteur AT-1, bloquant ainsi l'effet de l'angiotensine II au niveau du récepteur AT-1 de l'angiotensine. A titre d'exemples de ces composés, on peut citer le candésartan, le candésartan cilexétil, le prosartan, l'irbésartan, le losartan, le sel potassique du losartan, l'olmésartan, le telmisartan, ou le valsartan. Ceux-ci sont utilisés dans les associations selon la présente invention à des doses thérapeutiques efficaces.

Selon un autre mode de réalisation préféré de la présente invention, les compositions comprennent des quantités thérapeutiques efficaces d'antagonistes de l'aldostérone en une seule prise journalière, tels que la spironolactone, ses dérivés, ou ses métabolites selon les posologies précédemment prescrites en association avec une quantité thérapeutique efficace d'inotrope ou inodilatateur tel que par exemple le pimobendane ou le levosimendane. Le pimobendane correspond au 4,5-dihydro-6-[2-(4-methoxyphenyl)-1H-benzimidazol-5-yl]-5-methyl-3(2H)-pyridazone dont la structure chimique est comme suit :

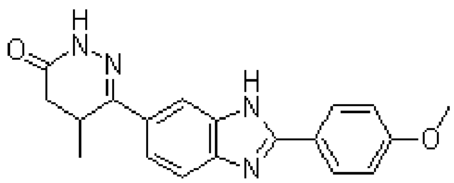

Le pimobendane est décrit entre autres dans les brevets US 4,361,563 et EP008391 et est commercialisé sous le nom Vetmedin® par Boehringer Ingelheim. Par ses mécanismes d'action (sensibilisateur calcique et inhibiteur de la phosphodiestérase III), il est inotrope positif (augmentation de la contractilité), lusitrope positif (amélioration de la relaxation) et vasodilatateur artériel (diminution de la postcharge), veineux (diminution de la précharge) et coronarien (amélioration de l'oxygénation du myocarde). Son action inotrope positive (liée à une augmentation de l'affinité de la troponine pour le calcium) s'exerce sans augmentation de la consommation énergétique du myocarde. L'effet vasodilatateur est intense et direct (par inhibition de la dégradation de l'AMPc dans la cellule musculaire lisse des vaisseaux). Les doses thérapeutiques efficaces de pimobendane administrées par voie orale avec les compositions selon l'invention. Par exemple, ces doses peuvent être d'environ 0,25 à 2 mg/kg/jour, et de préférence d'environ 0,5 mg/kg/jour.

On peut citer également le levosimendane qui est décrit comme inodilatateur dans le brevet européen EP383449, et correspond au [[4-(1,4,5,6,-tétrahydro-4-méthyl-6-oxo-3-pyridazinyl) phényl] hydrazono] propane nitrile dont la structure chimique comme suit :

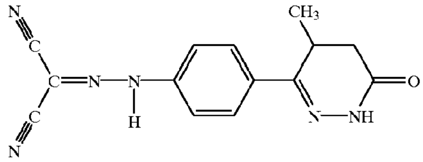

Le levosimendane peut être administré oralement ou par injection. Les doses thérapeutiques efficaces de levosimendane utilisées dans les compositions selon la présente invention. Ces doses peuvent être par exemple comprises entre 0,025 et 0,5 mg/kg/jour selon la voie d'administration choisie. A titre d'exemples, lorsqu'elles sont administrées par voie orale, les doses thérapeutiques efficaces peuvent être de 0.1 mg/kg à 0.2 mg/kg en deux prises (0.05 à 0.1 mg/kg matin et soir).

De manière encore plus préférentielle, les compositions comprennent des quantités thérapeutiques efficaces d'antagonistes de l'aldostérone, tels que la spironolactone, ses dérivés, ou ses métabolites administrées selon les posologies précédemment décrites en association avec des quantités thérapeutiques efficaces d'inotropes ou inodilatateurs tels que le pimobendane ou le levosimendane et d'IEC, tel que le benazepril ou l'enalapril. Ces compositions sont particulièrement efficaces pour traiter et/ou prévenir les MVD des chiens, les CMD des chiens et des chats ainsi que les CMH des chats.

Les compositions d'antagonistes de l'aldostérone tels que la spironolactone, ses dérivés, ou ses métabolites selon l'invention peuvent être administrées avec des vasodilatateurs, tel que le nitroprusside sodium, la nitroglycerine, ou l'isosorbide nitrate, et/ou des diurétiques tels que le furosémide, le bumétanide, le torasémide, ou les thiazidiques tels que le chlorothiazide ou l'hydrochlorothiazide. Les diurétiques sont utilisés en cas de signes congestifs marqués (oedème pulmonaire, ascite...), à la dose la plus faible nécessaire. Lorsque les compositions selon l'invention sont administrées en association avec un diurétique, celui-ci est de préférence le furosémide à une dose de 4-8 mg/kg/jour et est également administré.

Les compositions d'antagonistes de l'aldostérone tels que la spironolactone, ses dérivés, ou ses métabolites précédemment décrites peuvent en outre être administrées avec d'autres traitements usuels de l'insuffisance cardiaque tels que les digitaliques dont la digoxine en tant que thérapie standard de l'insuffisance cardiaque notamment pour le traitement des arythmies supraventriculaires, en particulier de la fibrillation atriale.

Les doses quotidiennes de ces traitements usuels de l'insuffisance cardiaque sont adaptées à chacun des mammifères non humains atteints d'insuffisance cardiaque, et traités avec les compositions selon la présente invention.

Les compositions sont utiles pour traiter et/ou prévenir les maladies valvulaires dégénératives (MVD) chez le chien, la cardiomyopathie dilatée (CMD) chez le chien et le chat et la cardiomyopathie hypertrophique (CMH) chez le chat. Egalement, elles sont particulièrement appropriées pour le traitement des animaux non humains atteints d'insuffisance cardiaque.

On entend par dose thérapeutique efficace ou active, une quantité susceptible de rétablir le rapport sodium potassium plasmatique et/ou d'induire un effet thérapeutique suffisant et ainsi permettre une réduction du taux de mortalité et/ou de morbidité important. Egalement, on entend par dose thérapeutique active, une quantité de chacun des ingrédients actifs susceptible de produire en combinaison un effet thérapeutique suffisant et ainsi permettre une réduction de la mortalité et/ou de la morbidité.

Selon la présente invention, le risque de mortalité observée est réduit d'un pourcentage d'au moins 50 %. De manière plus préférentielle, le pourcentage de réduction du risque de mortalité est compris entre environ 80% et 50%, 80% et 55%, 80% et 60%, 80% et 65%, 80% et 70%, ou entre 80% et 75%, et est par exemple d'environ 80%, 73%, 67%, 65 %, ou 59%. L'effet protecteur est particulièrement important pour les animaux atteints d'insuffisance cardiaque incluant les stades débutants. Le risque de morbi-mortalité est réduit d'au moins 40% ou d'au moins 46%.

Ces compositions de spironolactone, ses dérivés, ou ses métabolites en administration unique par 24h et/ou en association avec des quantités thérapeutiques efficaces d'IEC, d'antagonistes du récepteur AT-1 de l'angiotensine II (ARA-II ou sartans), de digitaliques, d'inotropes, d'inodilatateurs, de diurétiques, de vasodilatateurs, de béta bloquants et/ou d'antagonistes calciques sont efficaces pour le traitement et/ou la prévention des insuffisances cardiaques chez les animaux mammifères non humains. Les compositions selon la présente invention sont particulièrement efficaces pour le traitement des insuffisances cardiaques congestives à régurgitation valvulaire. En effet, il a été démontré que les chiens souffrant notamment d'insuffisance cardiaque congestive due à une régurgitation valvulaire auxquels sont administrés les compositions de spironolactone, ses dérivés, ou ses métabolites selon les posologies précédemment décrites et en seule prise en association avec une thérapie standard, présentent une durée de vie plus longue que les chiens ayant reçu une thérapie standard seule. Cette posologie peut être maintenue en traitement long terme par exemple sur 15 mois ou 36 mois. En effet, à long terme, il a été démontré que l'insuffisance cardiaque des sujets animaux non humains s'est moins aggravée que celle des chiens ayant seulement reçu une thérapie standard.

Les animaux mammifères non humains peuvent recevoir de préférence des doses thérapeutiquement efficaces

de spironolactone et de benazépril. Ceux-ci peuvent être administrés de manière séquentielle ou simultanée selon toutes voies d'administration bien connues dans le domaine et adaptées au traitement de chacun des animaux, telles que les voies nasale, orale et parentérale. Les méthodes selon l'invention permettent le traitement de sujets atteints d'insuffisance cardiaque, notamment les animaux de compagnie tels que par exemple, les chiens, les chats, ou les chevaux. Lorsque les méthodes selon ce mode de réalisation sont destinées au traitement des chiens atteints d'insuffisance cardiaque, une dose quotidienne d'antagoniste du récepteur l'aldostérone comprise entre 0,88 et 5 mg/kg/jour, et de préférence d'environ 2 mg/kg/jour, et une dose quotidienne d'inhibiteur d'enzyme de conversion de l'angiotensine comprise entre 0,1 et 0,6 mg/kg/jour et de préférence d'environ 0,25 mg/kg/jour sont administrées. Lorsque les méthodes selon ce mode de réalisation sont destinées au traitement des chats atteints d'insuffisance cardiaque, une dose quotidienne d'antagoniste du récepteur l'aldostérone comprise entre 0,88 et 5 mg/kg/jour, et de préférence d'environ 2 mg/kg/jour, et une dose quotidienne d'inhibiteur d'enzyme de conversion de l'angiotensine comprise entre 0,1 et 0,6 mg/kg/jour, et de préférence d'environ 0,25 mg/kg/jour sont alors administrées. Enfin, lorsque les méthodes selon ce mode de réalisation sont destinées au traitement des chevaux atteints d'insuffisance cardiaque, on administre une dose quotidienne d'antagoniste du récepteur l'aldostérone comprise entre 0,88 et 5 mg/kg/jour, et de préférence d'environ 2 mg/kg/jour, et une dose quotidienne d'inhibiteur d'enzyme de conversion de l'angiotensine est comprise entre 0,1 et 0,6 mg/kg/jour, et de préférence d'environ 0,25 mg/kg/jour. Comme cela est indiqué précédemment, les administrations peuvent être effectuées de manière simultanée ou séquentielle.

Les compositions ou médicaments vétérinaires selon la présente invention peuvent se présenter sous toutes formes appropriées selon les modes d'administration souhaités par exemple par voie nasale, orale, intradermique, cutanée ou parentérale. Elles peuvent donc être sous une forme d'une solution ou d'une suspension liquide nasale, orale ou injectable, ou sous la forme solide ou semi-solide, de poudres, de comprimés, de capsules, de granules, de dragées, de gélules, de sprays, de cachets, de pilules, de tablettes, de pâtes, d'implants ou de gels.

Selon les formulations des compositions et médicaments utilisées, celles-ci peuvent comprendre en outre des ingrédients conventionnellement utilisés en pharmacie pour la préparation des formulations liquides ou solides pour une administration par voie nasale, orale, intradermique, cutanée ou parentérale. Ainsi les compositions selon l'invention peuvent comprendre selon le type de formulations, un agent d'écoulement, un lubrifiant et tout excipient de masse convenable, tel que le lactose, la cellulose ou les amidons. Comme lubrifiant, on peut utiliser l'acide stéarique, le stéarate de magnésium, la L-leucine ou, par exemple, le tribéhénate de glycérol. Comme agent de désintégration, on peut utiliser le carboxyméthylamidone sodique, la carboxyméthylcellulose sodique réticulée ou, par exemple, la polyvinylpyrrolidone réticulée. Comme agent d'écoulement, on peut utiliser la silice pure ou le dioxyde de silicium colloïdal.

Les formes orales peuvent être sous formes de comprimés à dissolution instantanée ou effervescentes obtenues en ajoutant un couple effervescent à la composition selon l'invention, ou encore sous forme de comprimés recouverts d'un enrobage. Comme couple effervescent, on peut utiliser, l'acide tartrique et le bicarbonate de sodium ou l'acide citrique et le bicarbonate de sodium.

Lorsque les compositions se présentent sous la forme de comprimés, ceux-ci sont par exemple des comprimés de spironolactone de 10mg, 40mg, ou 80mg. Les comprimés sont sécables pour permettre de les couper et de respecter ainsi la posologie selon l'invention en une seule prise journalière.

Les préparations injectables sont préparées par mélange de quantités thérapeutiquement efficaces d'antagonistes de l'aldostérone et par exemple d'IEC et/ou un inotrope, voire un inodilatateur, avec un régulateur de pH, un agent tampon, un agent de mise en suspension, un agent de solubilisation, un stabilisant, un agent de tonicité et/ou un conservateur, et par transformation du mélange en une injection intraveineuse, sous-cutanée, intramusculaire ou perfusion, selon un procédé classique. Le cas échéant, les préparations injectables peuvent être lyophilisées selon un procédé classique. Des exemples d'agents de mise en suspension englobent la méthylcellulose, le polysorbate 80, l'hydroxyéthylcellulose, la gomme de xanthane, la carboxyméthycellulose sodique et le monolaurate de sorbitane polyéthoxylé. Des exemples d'agent de solubilisation englobent l'huile de ricin solidifiée par du polyoxyéthylène, le polysorbate 80, le nicotinamide, le monolaurate de sorbitane polyéthoxylé, le macrogol et l'ester éthylique d'acide gras d'huile de ricin. En outre, le stabilisant englobe le sulfite de sodium, le métalsulfite de sodium et l'éther, tandis que le conservateur englobe le p-hydroxybenzoate de méthyle, le p-hydroxybenzoate d'éthyle, l'acide sorbique, le phénol, le crésol et le chlorocrésol. Un exemple d'agent de tonicité est le mannitol. Lors de la préparation des solutions ou suspensions injectables, il est souhaitable de veiller à ce qu'elles soient isotoniques au sang.

La présente invention a par ailleurs pour objet un kit à usage vétérinaire destiné au traitement de sujets mammifères non humains atteints d'insuffisance cardiaque, ayant au moins un compartiment pour un conditionnement stérile ou non, séparé ou non, pour une administration simultanée ou séquentielle des doses quotidiennes d'antagoniste de l'aldostérone seul en en association avec un traitement standard de l'insuffisance cardiaque, tel que par exemple un IEC, un antagoniste du récepteur AT-1 de l'angiotensine II (ARA-II ou sartans), un digitalique, un inotrope, un inodilatateur, un diurétique, un vasodilatateur, un béta bloquant et/ou un antagoniste calcique. Le ou les compartiments peuvent ainsi comprendre une dose quotidienne d'antagoniste de l'aldostérone supérieure à 1mg/kg/jour et inférieure à 5 mg/kg/jour, comprise entre 1,5 et 5 mg/kg/jour, 1,8 et 5 mg/kg/jour, 1,5 et 4 mg/kg/jour, 1,5 et 3 mg/kg/jour, de préférence comprise

entre 2 et 5 mg/kg/jour, et de manière encore plus préférentielle d'environ 2 mg/kg/jour ou 4 mg/kg/jour, et/ou une dose quotidienne d'un inhibiteur d'enzyme de conversion de l'angiotensine d'environ 0,1 à 0,6 mg/kg/jour, de préférence d'environ 0,25 mg/kg/jour pour le benazépril et 0,5mg/kg/jour pour l'énalapril, et/ou environ 0,25-2mg/kg/jour de pimobendane, et/ou environ 0,025-0,5mg/kg/jour de levosimendane.

Le kit selon ce mode de réalisation facilite l'administration des posologies prescrites à chaque sujet, de manière simultanée ou séquentielle, et au moins une fois par jour. Egalement, le kit selon l'invention comprend un conditionnement stérile ou non stérile de l'antagoniste de l'aldostérone avec sans thérapie standard, adapté pour leur administration orale, nasale, intradermique, cutanée, ou parentérale, ainsi que les moyens permettant l'administration de ces formulations. Enfin, les kits selon la présente invention, comprennent en outre un emplacement pour une fiche d'instructions concernant le mode opératoire et le mode d'administration des formulations.

Les antagonistes de l'aldostérone et les traitements standards de l'insuffisance cardiaque, tels que des IEC, des antagonistes du récepteur AT-1 de l'angiotensine II (ARA-II ou sartans), des digitaliques, des inotropes, des inodilatateurs, des diurétiques, des vasodilatateurs, des béta bloquants et/ou des antagonistes calciques sont tels que décrits précédemment pour les compositions, et peuvent avantageusement être administrés simultanément ou séquentiellement, par voie nasale, orale, intradermique, cutanée ou parentérale. Egalement, les doses appropriées pour chacun des animaux mammifères non humains à traiter sont telles que décrites précédemment par exemple pour le chien, le chat ou le cheval.

La présente invention a en outre pour objet l'utilisation de quantités thérapeutiques efficaces d'un antagoniste de l'aldostérone tel que la spironolactone, ses dérivés, ou ses métabolites en vue de la préparation d'un médicament vétérinaire pour la prévention et/ou le traitement des animaux non humains atteints d'insuffisance cardiaque non décompensée, sans provoquer d'effets secondaires d'hyperkaliémie, dans lequel ledit antagoniste de l'aldostérone est administré en une dose quotidienne supérieure à 1mg/kg/jour et inférieure à 5 mg/kg/jour, comprise entre 1,5 et 5 mg/kg/jour, 1,8 et 5 mg/kg/jour, 1,5 et 4 mg/kg/jour, 1,5 et 3 mg/kg/jour, de préférence comprise entre 2 et 5 mg/kg/jour, et de manière encore plus préférentielle d'environ 2 mg/kg/jour ou 4 mg/kg/jour en une seule prise. De préférence, les compositions selon la présente invention sont administrées aux stades débutants de l'insuffisance cardiaque, notamment avant l'apparition des oedèmes chez les animaux non humains ainsi traités.

De préférence, la présente invention a en outre pour objet l'utilisation de quantités thérapeutiques efficaces d'un antagoniste de l'aldostérone en association avec une thérapie standard de l'insuffisance cardiaque, telle que des IEC, des antagonistes du récepteur AT-1 de l'angiotensine II (ARA-II ou sartans), des digitaliques, des inotropes, des inodilatateurs, des diurétiques, des vasodilatateurs, des béta bloquants et/ou des antagonistes calciques en vue de la préparation d'un médicament vétérinaire destiné à réduire le taux de mortalité et/ou de morbidité des sujets animaux mammifères non humains atteints d'insuffisance cardiaque, sans provoquer d'effets secondaires d'hyperkaliémie, dans lequel ledit antagoniste de l'aldostérone est administré en une dose quotidienne supérieure à 1mg/kg/jour et inférieure à 5 mg/kg/jour, comprise entre 1,5 et 5 mg/kg/jour, 1,8 et 5 mg/kg/jour, 1,5 et 4 mg/kg/jour, 1,5 et 3 mg/kg/jour, de préférence comprise entre 2 et 5 mg/kg/jour, et de manière encore plus préférentielle d'environ 2 mg/kg/jour ou 4 mg/kg/jour en une seule prise.

Les antagonistes de l'aldostérone sont tels que précédemment décrits et sont de préférence choisis parmi la spironolactone ou l'éplérénone ou des dérivés, des métabolites de ces composés. Les thérapies standards de l'insuffisance cardiaque sont telles que précédemment décrites et peuvent être choisies parmi les IEC, les antagonistes du récepteur AT-1 de l'angiotensine II (ARA-II ou sartans), les digitaliques, les inotropes, les inodilatateurs, les diurétiques, les vasodilatateurs, les béta bloquants et/ou les antagonistes calciques. Les IEC sont par exemple, l'alacepril, le benazepril, le captopril, le cilazapril, le delapril, l'enalapril, l'enalaprilat, le fosinopril, le fosinoprilat, l'imidapril, l'idrapril, le lisinopril, le perindopril, le quinapril, le ramipril, l'acétate de saralasin, le perindropilat, le temocapril, le trandolapril, le ceranapril, le moexipril, le quinaprilat, le spirapril, un sel ou un ester pharmaceutiquement acceptable de ces composés. De préférence, le benazépril, ses dérivés tels que le chlorhydrate de benazépril, et/ou l'enalapril sont utilisés.

Les utilisations selon ce mode de réalisation permettent la préparation de médicaments à usage vétérinaire pour le traitement des animaux mammifères non humains, notamment les animaux de compagnie, tels que par exemple les chiens, les chats, ou les chevaux. Ces utilisations permettent notamment la préparation de médicaments à usage vétérinaire pour le traitement des MVD des chiens, des CMD des chiens et des chats, et des CMH chez des chats.

Lorsque les médicaments vétérinaires sont utilisés pour le traitement des chiens, la dose quotidienne d'antagoniste de l'aldostérone utilisée est supérieure à 1mg/kg/jour et inférieure à 5 mg/kg/jour, comprise entre 1,5 et 5 mg/kg/jour, 1,8 et 5 mg/kg/jour, 1,5 et 4 mg/kg/jour, 1,5 et 3 mg/kg/jour, de préférence comprise entre 2 et 5 mg/kg/jour, et de manière encore plus préférentielle d'environ 2 mg/kg/jour ou 4 mg/kg/jour en une seule prise. La dose quotidienne d'inhibiteur d'enzyme de conversion de l'angiotensine est comprise entre 0,1 et 0,6 mg/kg/jour, et de préférence d'environ 0,25 mg/kg/jour pour le benazépril, et 0,5 mg/kg/jour pour l'énalapril. Ceux-ci peuvent également comprendre une dose thérapeutique efficace d'un inotrope tel que le pimobendane ou le levosimendane. Par exemple, une dose thérapeutique efficace peut être comprise entre 0,25-2mg/kg/jour pour le pimobendane et de 0,025-0,5mg/kg/jour pour le levisomendane.

Comme cela est décrit précédemment, les compositions vétérinaires et les médicaments destinés au traitement des animaux de compagnie peuvent se présenter sous toutes formes appropriées selon les modes d'administration souhaités par exemple par voie nasale, orale, intradermique, cutanée ou parentérale. Elles peuvent donc être sous une forme d'une solution liquide orale ou injectable, sous forme d'une suspension ou sous forme solide ou semi-solide, de poudres, de comprimés, de capsules, de granules, de dragées, de gélules, de sprays, de cachets, de pilules, de tablettes, de pâtes, des implants ou de gels.

Encore un autre objet de la présente invention consiste en une méthode de traitement et/ou de prévention des animaux non humains atteints d'insuffisance cardiaque non décompensée, sans provoquer d'effets secondaires d'hyperkaliémie non réversible, comprenant l'administration de compositions d'antagoniste de l'aldostérone, tel que par exemple la spironolactone ses dérivés, ou ses métabolites, en une seule prise par jour, et selon les posologies décrites ci-dessus. Les méthodes de traitement selon l'invention consistent de préférence à administrer lesdites compositions aux stades débutants de l'insuffisance cardiaque, notamment avant l'apparition des oedèmes chez les animaux non humains ainsi traités.

La présente invention a également pour objet une méthode de traitement des animaux non humains atteints d'insuffisance cardiaque, notamment des MVD chez le chien, la CMD du chien et du chat, et la CMH du chat, comprenant l'administration de compositions d'antagoniste de l'aldostérone, tel que par exemple la spironolactone, ses dérivés, ou ses métabolites, en une seule prise par jour, et selon les posologies de la présente invention en association avec une thérapie standard de l'insuffisance cardiaque.

La présente invention a en outre pour objet une méthode de réduction du taux de mortalité et/ou de morbidité des sujets animaux mammifères non humains atteints d'insuffisance cardiaque comprenant l'administration de quantités thérapeutiques efficaces d'un antagoniste de l'aldostérone seul ou en combinaison avec les IEC, les antagonistes du récepteur AT-1 de l'angiotensine II (ARA-II ou sartans), les digitaliques, les inotropes, les inodilatateurs, les diurétiques, les vasodilatateurs, les béta bloquants et/ou les antagonistes calciques. Selon l'invention, l'antagoniste de l'aldostérone est administré en une dose quotidienne supérieure à 1mg/kg/jour et inférieure à 5 mg/kg/jour, comprise entre 1,5 et 5 mg/kg/jour, 1,8 et 5 mg/kg/jour, 1,5 et 4 mg/kg/jour, 1,5 et 3 mg/kg/jour, de préférence comprise entre 2 et 5 mg/kg/jour, et de manière encore plus préférentielle d'environ 2 mg/kg/jour ou 4 mg/kg/jour en une seule prise, et la réduction du risque de mortalité observée est d'au moins 50%. De manière plus préférentielle, la réduction du risque de mortalité est comprise entre environ 80% et 50%, entre 80% et 55%, entre 80% et 60%, entre 80% et 65%, entre 80% et 70%, ou entre 80 et 75% et est par exemple d'environ 80%, 73%, 67%, 65 %, ou 59%. L'effet protecteur est particulièrement important pour des animaux atteints d'insuffisance cardiaque incluant des stades débutants de la pathologie. Selon ce mode de réalisation, le risque de morbi-mortalité est réduit d'au moins 40% ou d'au moins 46%. Par ailleurs, les méthodes de traitement selon l'invention ne provoquent pas d'effets secondaires d'hyperkaliémie. Selon les méthodes de traitement de l'invention, l'antagoniste de l'aldostérone peut être utilisé seul ou en association avec une thérapie standard de l'insuffisance cardiaque, qui est alors administré en une dose thérapeutiquement efficace d'environ 0,1 à 0,6 mg/kg/jour, tel que de préférence d'environ 0,25 mg/kg/jour pour le benazépril et/ou d'environ 0,5 mg/kg/jour d'énalapril, et/ou une dose de 0,25-2mg/kg/jour de pimobendane et/ou une dose de 0,025-0,5mg/kg/jour de levosimendane.

Les antagonistes de l'aldostérone, ainsi que les thérapies standards de l'insuffisance cardiaque, tels que précédemment décrits peuvent être utilisés dans la méthode de la présente invention. De préférence, la spironolactone ou l'épléránone, ou les dérivés, les métabolites, de ces composés est administré en tant qu'antagoniste de l'aldostérone selon les méthodes de traitement de la présente invention. Lorsque la spironolactone, ses dérivés, ou ses métabolites est administrée en association avec une thérapie standard, par exemple avec des IEC, ceux-ci peuvent être choisis parmi l'alacepril, le benazépril, le captopril, le cilazapril, le delapril, l'énalapril, l'énalaprilat, le fosinopril, le fosinoprilat, l'imidapril, l'idrapril, le lisinopril, le perindopril, le quinapril, le ramipril, l'acétate de saralasin, le perindropilat, le temocapril, le trandolapril, le ceranapril, le moexipril, le quinaprilat, le spirapril, les sel ou les esters pharmaceutiquement acceptables de ces composés.

Les animaux mammifères non humains reçoivent de préférence des doses thérapeutiques efficaces de spironolactone, ses dérivés, ou ses métabolites et de benazépril ou de l'énalapril et/ou de pimobendane et/ou le levosimendane. Ceux-ci peuvent être administrés de manière séquentielle ou simultanée selon toutes voies d'administration bien connues dans le domaine et adaptées au traitement de chacun des animaux, telles que les voies nasale, orale, intradermique, cutanée et parentérale.

Les méthodes selon l'invention permettent le traitement de sujets atteints d'insuffisance cardiaque, notamment les animaux de compagnie tels que par exemple, les chiens, les chats, ou les chevaux.

Lorsque les méthodes selon ce mode de réalisation sont destinées au traitement des chiens atteints de MVD ou de CMD, une dose quotidienne d'antagoniste l'aldostérone comprise entre 0,88 et 5 mg/kg/jour, 1-5mg/kg/jour, 1,5-5 mg/kg/jour, 1,8-5 mg/kg/jour, ou 2-5 mg/kg/jour, et de préférence d'environ 2 mg/kg/jour en une seule prise, et en association avec une thérapie standard tel que IEC à raison de 0,1 et 0,6 mg/kg/jour et/ou un inotrope (voire un inodilatateur), tel que le pimobendane. Une dose thérapeutique efficace de pimobendane peut être par exemple de 0,25-2 mg/kg/jour.

Lorsque les méthodes selon ce mode de réalisation sont destinées au traitement des chats atteints de CMD ou de CMH, une dose quotidienne d'antagoniste de l'aldostérone supérieure à 1mg/kg/jour et inférieure à 5 mg/kg/jour, comprise entre 1,5 et 5 mg/kg/jour, 1,8 et 5 mg/kg/jour, 1,5 et 4 mg/kg/jour, 1,5 et 3 mg/kg/jour, de préférence comprise entre 2 et 5 mg/kg/jour, et de manière encore plus préférentielle d'environ 2 mg/kg/jour ou 4 mg/kg/jour en une seule prise peut être administrée seule ou en association par exemple avec une dose d'IEC comprise entre 0,1 et 0,6 mg/kg/jour, et/ou une dose de pimobendane de 0,25-2 mg/kg/jour. Les chats atteints de CMH sont traités, selon l'invention par l'administration d'une dose quotidienne d'antagoniste de l'aldostérone supérieure à 1mg/kg/jour et inférieure à 5 mg/kg/jour, comprise entre 1,5 et 5 mg/kg/jour, 1,8 et 5 mg/kg/jour, 1,5 et 4 mg/kg/jour, 1,5 et 3 mg/kg/jour, de préférence comprise entre 2 et 5 mg/kg/jour, et de manière encore plus préférentielle d'environ 2 mg/kg/jour ou 4 mg/kg/jour en une seule prise peut être administrée seule ou en association par exemple avec une dose d'IEC comprise entre 0,1 et 0,6 mg/kg/jour.

Ces administrations peuvent être effectuées séparément ou en association de façon simultanée ou séquentielle. Egalement, selon les méthodes de l'invention, les compositions administrées telles que précédemment décrites peuvent se présenter sous diverses formes telles que par exemple sous formes de solutions liquides, sous forme de suspensions, solides ou semi-solides, sous forme de poudres, de comprimés, de capsules, de granules, de dragées, de gélules, de sprays, de cachets, de pilules, de tablettes, de pâtes, d'implants ou de gels.

## EXEMPLES

### Exemple 1

Des études de pharmacocinétiques de la spironolactone en administration orale ont été réalisées sur différentes espèces, telles que le rat, le chien, et le singe en utilisant de la spironolactone marquée (22-$^{14}$C spironolactone). Les résultats ont été présentés sous forme de courbes logarithmiques dans la Figure 1, et montrent un pourcentage de radioactivité dans le plasma élevé chez le rat (66%) et le chien (76%) et plus bas chez le singe (33%) après 4 h d'administration par voie orale de spironolactone.

Il a été découvert selon la présente invention que contrairement au doses utilisées pour les traitements des patients humains, la dose optimale de spironolactone pour le traitement de l'insuffisance cardiaque chez les animaux de compagnie tels que le chien, le chat, le cheval était voisine de 2mg/kg/jour. Les changements des valeurs logarithmiques ($[Na^+]_{urinary}$ x $10/[K^+]_{urinary}$) induites par l'aldostérone ont été mesurées après traitement par la spironolactone.

Pour réaliser ces études, des chiens sains de race beagle (n=15) âgés de moins d'un an, et pesant entre 11,9 et 14,3 kg au début de l'étude ont été utilisés. Ils ont été préalablement tatoués afin de pouvoir les identifier facilement et sont placés dans des box individuels en acier inoxydable. La température de la pièce a été maintenue entre 17-21°C, et l'humidité entre 45 et 65%. Les pièces ont été par ailleurs éclairées pendant 12 heures puis placées dans l'obscurité pendant 12 heures.

Le modèle d'évaluation de l'activité anti-aldostérone tel que décrit par Hofman L.M et al. (1975, The Journal of Pharmacology and Experimental Therapeutics. 194, 450-456) a été utilisé. Selon ce modèle expérimental, l'aldostérone a été injectée immédiatement après l'administration orale de comprimés de spironolactone (10mg, 40mg, ou 80mg). Une étude préliminaire a été effectuée afin de déterminer la valeur de l'$ED_{80}$ pour l'effet natriurétique de l'aldostérone seule. Tous les animaux avaient été dosés de manière successive avec un vecteur (groupe témoin) puis en présence d'aldostérone administrée à des doses croissantes de 0, 3 μg/kg; 1 μg/kg et 3 μg/kg. Une période d'élimination d'au moins 48 heures a été laissée entre chaque administration. La dose optimale d'aldostérone de 3 μg/kg a ainsi été sélectionnée à la fin de cette étude préliminaire, et constitue une dose de référence permettant d'établir des corrélations avec l'aldostéronemie observée chez les chiens souffrant d'insuffisance cardiaque.

Les chiens décrits ci-dessus ont été affectés à différents groupes de traitement (A, B, C, D, et E) et une période de repos a été laissée entre les traitements.

Les doses de spironolactone testées ont été administrées par gavage lors d'une seule administration. Les comprimés de spironolactone ont été divisés en deux parties et un maximum de trois comprimés a été administré au chien afin d'atteindre la dose. Un groupe A de contrôle négatif n'a reçu que l'excipient pharmaceutique. Le groupe B de contrôle positif n'a reçu que 3 μg/kg d'aldostérone. Le groupe C a reçu 3 μg/kg d'aldostérone et 0,88 mg/kg de spironolactone. Le groupe D a reçu 3 μg/kg d'aldostérone et 2 mg/kg de spironolactone. Le groupe E a reçu 3 μg/kg d'aldostérone et 8 mg/kg de spironolactone. Les animaux avaient été conservés dans leur box individuels à jeun et leurs urines étaient collectées environ 16h avant le traitement. 200g de nourriture a été ensuite donnée immédiatement après le traitement.

Des échantillons de sang (5ml) ont été prélevés de la veine céphalique immédiatement après administration de spironolactone, puis à 3, 6, 9, 12 et 24 h après traitement. Les tubes ont été centrifugés rapidement à 1500 rpm pendant 10 minutes à une température de +4°C ± 2°C), et le plasma a été réparti dans deux tubes de propylène (1,2mL). Les échantillons ont été congelés et conservés dans l'obscurité à environ -80 °C. Le jour du test, les vessies des animaux

ont été vidées par cathérisation, puis à 6 h et à 24h après le traitement. Egalement, l'urine a été récupérée au cours des périodes de temps allant de To- $T_{6h}$; $T_{6h}$-$T_{12h}$ et $T_{12h}$-$T_{24h}$.

Les concentrations plasmatiques d'aldostérone ont été ensuite mesurées par radioimmunoassay sur phase solide (Coat-A-Count® Aldosterone) basée sur l'utilisation d'un anticorps spécifique de l'aldostérone immobilisé sur la paroi du tube de polypropylène. $^{125}$I-aldosterone marqué rentre en compétition avec l'aldostérone circulant de l'échantillon (200 μL) pour la fixation sur l'anticorps. Après calibration et comptage, la quantité d'aldostérone présente dans l'échantillon a été ainsi déterminée après comptage. La fourchette était comprise entre 25 et 1200 pg/mL.

Les niveaux de sodium et de potassium dans les urines ont été déterminés à l'aide d'un osmomètre. Une méthode de détection par HPLC couplée à la détection UV a été utilisée pour mesurer les niveaux plasmatiques de spironolactone et des métabolites (7α-thiométhyl-spirolactone et canrénone). Selon cette méthode 500μL de plasma ont été mélangés avec de l'acétate d'éthyl(80/20,V/V) en tant que solvants. La spironolactone, la 7α-thiométhyl-spirolactone du 1,1,1 trichloroéthane et la canrénone ont été extraits par extraction liquide-liquide. Les composés et le standard interne (à savoir le méthyl-testostérone) ont été séparés sur colonne Kromasil C18. Les niveaux de quantification étaient de 10 μg/L pour tous les composés à +/- 14% de variation.

Les études de pharmacocinétique des concentrations plasmatiques de spironolactone et des métabolites ont été réalisées en utilisant un programme d'analyse par régression linéaire (Kinetica version 4.0, THERMO ELECTRON Corporation, USA).

La réponse $Na^+/K^+$ à l'administration de spironolactone a été évaluée en mesurant le rapport logarithmique ($[Na^+]_{urinary}$ x $10/[K^+]_{urinary}$) des concentrations urinaires de $Na^+/K^+$ collectées au temps 0, c'est-à-dire juste après l'administration de spironolactone et 6 h après l'administration de spironolactone. Aucune correction n'a été apportée pour rendre compte des niveaux de base logarithmiques de ($[Na^+]_{urinary}$ x $10/[K^+]_{urinary}$), et seul le modèle du $E_{max}$ model a été effectué à partir de l'administration de la spironolactone. La relation entre le rapport logarithmique ($[Na^+]_{urinary}$ x $10/[K^+]_{urinary}$) pendant les premières 6 heures de mesure de l'effet a été analysée en fonction de la dose de spironolactone selon le modèle sigmoïde classique $E_{max}$ Equation 3):

$$E_{\log(Na^+ * 10/K^+)} = E_0 + \frac{(\mathrm{E_{max}} - \mathrm{E_0}) \times Dose_{(spironolactone\ mg/kg)}}{ED_{50} \times Dose_{(spironolactone\ mg/kg)}} \qquad \text{Eq.3}$$

dans lequel $E_0$ est l'effet de base mesuré en tant que changement du log ($[Na^+]_{urinary}$ x $10/[K^+]_{urinary}$) pendant la période allant de 0 à 6 heures après administration de l'aldostérone administration seule (contrôle positif), $E_{max}$ est le maximum de réponse des niveaux de $Na^+$ et $K^+$ exprimé en termes de log ($[Na^+]_{urinary}$ x $10/[K^+]_{urinary}$) pendant la période allant de 0 à 6 heures après administration de spironolactone, $ED_{50}$ correspond à la quantité de spironolactone nécessaire pour atteindre 50% de la réponse maximale (à savoir moitié $_{\log([Na+]urinary \times 10/[K+])\ max}$), $E_{max}$-$E_0$ est la différence dans l'effet mesuré du log ($Na^+ * 10/K^+$) pendant la période allant de 0 à 6 heures après administration de spironolactone, $E_{log}$ (Na+ *10/K+) est l'effet en présence de spironolactone. La $Dose_{(spironolactone,\ mglkg)}$, $E_0$, $E_{log\ ([Na+]urinary\ x\ 10/[K+])\ max}$ and $ED_{50}$ ont été obtenues par régression non-linéaire, et n est le coefficient de Hill décrivant la relation dose-effet.

Une analyse statistique a été réalisée avec le logiciel STATGRAPHICS Plus version 4.1. (Manugestics, Inc., Rockville, Maryland, U.S.A.). Les résultats ont été présentés comme des moyennes ± SD;. Le $p<0.05$ est considéré comme étant significatif.

Le Tableau 3 ci-dessous présente les paramètres pharmacocinétiques de canrénone obtenus pour chaque dose de spironolactone de 0,8 mg/kg, 2mg/kg, et 8 mg/kg. La clairance apparente pour chaque dose *($Cl_{canrenone}$)* était de 26 ± 8 L/kg/h$^{-1}$.

Tableau 3:

| Paramètres | Doses (mg/kg) | | |
|---|---|---|---|
| | 0.8 | 2.0 | 8.0 |
| $AUC_{inf}$(μg.h.L$^{-1}$) | 426.6 ± 306.7 | 1099.0 ± 358.3 | 4794.2 ± 1393.4 |
| Cmax (μg/L) | 30.9 ± 18.3 | 74.7 ± 23.9 | 261.9 ± 53.8 |
| Tmax (h) | 5.0 ± 2.17 | 5.6 ± 2.8 | 6.0 ± 1.6 |
| Cmin (μg/L) | 15.926 ± 4.290 | 16.951 ± 8.380 | 177.048 ± 42.495 |
| Clairance (L/kg/h) | 26.1 ± 8.36 | 25.7 ± 8.0 | 23.0 ± 6.2 |

$AUC_{inf}$ = est l'aire totale sous la courbe de concentration de canrénone en fonction du temps calculé selon la règle trapézoïdale. Cmax est la concentration plasmatique maximale de canrénone; Tmax = est le temps où la concentration plasmatique de canrénone est maximale.

La Figure 2 représente un graphe semi-logarithmique de la concentration plasmatique de canrénone au cours du temps après administration orale de canrénone à des doses de 0,8 mg/kg, 2mg/kg et 8 mg/kg à 15 chiens. La concentration de canrénone a été détectée jusqu'à 5-6 h après administration. Ces trois courbes ont révélé un parallélisme des pentes terminales. Les valeurs de $AUC_{canrenone}$ étaient de 427 ± 307, 1099 ± 358 et 4794 ± 1393 μg.h.$L^{-1}$. Pour le Cmax, les valeurs correspondantes étaient de 30.9 ± 18.3, 74.7 ± 23.9 et 261.9 ± 53.8 μg/L.

Les doses 0,8 mg/kg, 2 mg/kg et 8 mg/kg de spironolactone ont été utilisées dans cette expérience, et la dose inhibitrice de l'effet de l'aldostérone sur le log ($[Na^+]_{urinary}$ x 10/$[K^+]_{urinary}$) a été observée pour les doses de 2 mg/kg et 8 mg/kg qui ont permis d'inverser complètement l'effet dans les premiers 6 heures et 12 heures après le dosage. L'aldostérone seule a diminué l'élimination de Na d'environ 65% et les niveaux urinaires de K ont été augmentés de 25%. La spironolactone a augmenté les rapports Na/K après traitement à l'aldostérone. En moyenne, il y a eu une réduction du log ($[Na^+]_{urinary}$ x 10/$[K^+]_{urinary}$) de 0.70 ± 0.22 à 1.14 ± 0.18 dans les échantillons d'urine collectés de 0 à 6 h après administration de spironolactone. Les réponses natriuretiques ont été complètement inversées à une dose de spironolactone de 2mg/kg, alors que la dose de 0.8mg/kg n'a aucun effet sur certains chiens. De plus d'autres augmentations de l'élimination de Na ont été observées à des doses plus élevées de spironolactone (8 mg/kg).

La Figure 3 représente la relation dose-effet entre les doses de spironolactone et le ratio ($[Na^+]_{urinary}$ x 10/ $[K^+]_{urinary}$). La Figure 3 montre l'existence d'une relation dose-effet en utilisant le modèle (Equation 3). La valeur $ED_{50}$ après administration de spironolactone était de 1,09 mg/kg. La valeur $E_{max}$ (à savoir l'effet maximum possible de la spironolactone) était de 1,089 et la valeur $E_0$ (groupe contrôle était de 0,527. Le $E_{max}$ - $E_0$ était de 0,5625 et correspondait à une amplitude de 100%. Par conséquent, la dose de spironolactone requise pour restaurer le rapport $Na^+/K^+$ dans les urines chez des chiens ayant reçu de l'aldostérone et donc en situation similaire d'insuffisance cardiaque était une dose d'environ 2 mg/kg (à savoir $E_{(2mg/kg)}$ - $E_0$ = 0,4933) et correspondait à la restauration de 88% de l'effet, alors que la dose de 0,8 mg/kg correspond à 57% (à savoir $E_{(0.8mg/kg)}$ - $E_0$ = 0,3233). La valeur $ED_{50}$ de 1,08 ± 0,28 mg/kg a été calculée à partir du modèle de $E_{max}$. La dose thérapeutique efficace susceptible de restaurer et normaliser les ratios était de 1,80 mg/kg par jour et correspond à une restauration de l'effet à 88%. Un maximum de chien a répondu positivement à un traitement avec de la spironolactone à raison d'environ 2 mg/kg et par jour.

**Exemple 2**

Des études cliniques ont été réalisées sur des chiens atteints d'insuffisance cardiaque pour évaluer les effets à longs termes (14-15 mois et 3 ans) de traitements comprenant la spironolactone à une dose de 2mg/kg/jour, ainsi qu'un IEC (tel que par exemple chlorhydrate de benazépril ou l'enalapril, etc..).

Des études cliniques multicentriques, randomisées, en double aveugle contre placebo ont été conduites. Un exemple d'étude a porté sur 221 chiens, le diagnostic d'insuffisance cardiaque reposant sur des symptômes de cardiomégalie ou de cardiomyopathie persistants après un premier traitement aux IEC. Sur 221 chiens, 109 ont reçu par voie orale journalière une dose de 2mg/kg/jour de spironolactone sous forme de comprimés de 10mg, 40mg, et/ou 80mg en association avec un IEC (par exemple le chlorhydrate de benazépril à une dose de 0,25 mg/kg/jour). Le groupe placébo de 112 chiens a reçu un placébo en association avec un IEC (par exemple le chlorhydrate de benazépril à une dose de 0,25 mg/kg/jour).

Pour apprécier les effets du traitement, les deux groupes ont été examinés à cinq reprises, à savoir, lors du premier jour de traitement (J1), puis lors du $84^{ième}$ jour (J84), du $162^{ième}$ jour (J162), du jour 252 (J252), et du $336^{ième}$ jour (J336). Cet examen a consisté en un examen clinique des chiens, des analyses d'urine et sanguine, et une radiographie. De plus, une échocardiographie a été conduite aux jours J1, J168, et J336.

L'efficacité et l'absence de toxicité de l'administration de doses de 2mg/kg/jour de spironolactone en association avec une thérapie standard à des chiens atteints d'insuffisance cardiaque ont été mis en évidence en comparaison avec les traitements standards seuls par les taux de mortalité et les taux mortalité-morbidité, ces derniers englobant l'ensemble des événements tels que la mort, l'euthanasie, ou la détérioration grave des chiens. Egalement, les effets du traitement ont été évalués sur les symptômes tels que la toux et la mobilité, ainsi que sur la prévention des symptômes tels que la dyspnée, l'oedème pulmonaire, la tolérance à l'effort. Les résultats obtenus sur 15 mois ont été mentionnés dans le Tableau 4.

Tableau 4 :

| Groupes traités | Nombre de chiens | Probabilité de « survie » (absence de morbi-mortalité) | Probabilité de survie (absence de mortalité) |
|---|---|---|---|
| Spironolactone (2mg/kg/jour) + IEC (par exemple le chlorhydrate de benazépril à 0,25mg/kg/jour) | 109 | 84% | 91% |
| Placébo + IEC (par exemple le chlorhydrate de benazépril à 0,25mg/kg/jour) | 112 | 67% | 74% |
| Total | 221 | | |

D'autres études cliniques à long terme ont été menées, et ont montré également une différence significative des probabilités de survie (absence de mortalité et/ou de morbidité) entre ces deux groupes de chiens traités spironolactone + thérapie standard et du groupe thérapie standard (groupe de référence). Les résultats de ces différentes études ont été présentés dans les Figures 2 à 6.

La Figure 4 illustre les probabilités de survie de chiens traités pendant une durée de 14 à15 mois de 91% contre 74% pour le groupe référence (p=0,011) ; la Figure 5 illustre les taux de mortalité obtenus à 14-15 mois, de 6% contre 20% pour le groupe référence (p=0,0029) ; la Figure 6 illustre les probabilités de survie de chiens traités pendant une durée de 3 ans de 80% contre 64% pour le groupe référence (p=0,017) ; la Figure 7 illustre les probabilités de survie de chiens traités dès le stade I de l'insuffisance cardiaque consécutive à une insuffisance valvulaire sur une durée d'environ 3,5 ans de 100% contre 53 %, (p=0,033) ; et la Figure 8 illustre les taux de morbidité-mortalité obtenus à 14-15 mois, de 11% contre 25%.

De plus, une amélioration des symptômes tels que la toux et de la mobilité a été observée dans le groupe des chiens spironolactone, ainsi que la prévention des symptômes de dyspnée, d'oedème pulmonaire, de tolérance à l'effort, et une détérioration significativement plus légère de la toux et des syncopes. Le groupe contrôle est apparu comme présentant une détérioration plus forte et plus fréquente de tous ces signes cliniques.

**Exemple 3**

Les mesures de concentration plasmatique de potassium (mmol/L) ont été effectuées pendant la durée des traitements. Il a été ainsi démontré que la dose quotidienne de spironolactone de 2mg/kg/jour qui est normalement une dose diurétique chez l'homme et le chien, n'entrainait aucune variation de la kaliémie ou seulement de faibles variations transitoires de kaliémie chez le chien. Les résultats des mesures de kaliémie réalisées lors des études cliniques précédemment décrites ont été donnés dans le Tableau 5 suivant. Seuls des cas sporadiques d'hyperkaliémie faible ou modérée ont été observés et ces événements étaient transitoires. En effet, ces événements d'hyperkaliémie n'ont été observés qu'à une ou deux occurrences lors des examens, et pour certaines étaient présentes au jour J1, avant le début du traitement.

Tableau 5 :

| | hyperkaliémie faible et transitoire (5.9 to 6.4 mmol/L) | Hyperkaliémie modérée et transitoire (6.5 to 7.5 mmol/L) |
|---|---|---|
| | GROUPE SPIRONOLACTONE | GROUPE PLACEBO |
| Etude clinique 3 mois (6 analyses pendant la durée de l'étude) | 5 | 21 0 |
| Etude clinique 2 mois (4 analyses pendant la durée de l'étude) | 3 | 112 |
| Etude clinique 12 mois (5 analyses pendant la durée de l'étude) | 3 | 212 |
| | 11/109 (10.1%) | 5/112 (4.5%) 3/109 (2.8%) 4/112 (3.6%) |

Egalement, la Figure 9 illustre une kaliémie stable dans les deux groupes de chiens traités et placébo, avec quelques rares cas d'hyperkaliémie faible ou modérée et de nature transitoire.

## Revendications

1. Une composition vétérinaire pour son utilisation dans traitement de sujets mammifères non humains atteints d'insuffisance cardiaque comprenant un antagoniste de l'aldostérone et un véhicule pharmaceutiquement acceptable, **caractérisée en ce que** ledit antagoniste de l'aldostérone est présent en une dose thérapeutique efficace comprise entre 2 et 5 mg/kg/jour.

2. Composition vétérinaire selon la revendication 1, **caractérisée en ce que** ledit antagoniste de l'aldostérone est présent en une dose thérapeutique efficace d'environ 2 mg/kg/jour.

3. Composition vétérinaire selon la revendication 1, **caractérisée en ce que** ledit antagoniste de l'aldostérone est présent en une dose thérapeutique efficace d'environ 4 mg/kg/jour.

4. Composition vétérinaire selon l'une quelconque des revendications précédentes, **caractérisée en ce que** ledit antagoniste de l'aldostérone est administré en une seule prise.

5. Composition vétérinaire selon l'une quelconque des revendications précédentes, **caractérisée en ce que** ledit antagoniste de l'aldostérone est choisi parmi les composés de type spironolactone, tels que la spironolactone, l'éplérénone ou les métabolites de ces composés.

6. Composition vétérinaire selon la revendication 5, **caractérisée en ce que** lesdits métabolites sont choisis parmi la canrénone, l'acide canrénoique, la 15β-OH canrénone, la 21-OH canrénone, le canrénoate de potassium, la 7α-thio-spironolactone, la 7α-thiométhylspironolactone, ou la 6-β-hydroxy-7-α-thiométhylspironolactone.

7. Composition vétérinaire selon l'une quelconque des revendications précédentes pour son utilisation dans le traitement de sujets mammifères non humains atteints d'insuffisance cardiaque congestive.

8. Composition vétérinaire selon la revendication 7, pour son utilisation dans le traitement de sujets mammifères non humains atteints de régurgitation valvulaire et/ou de maladie valvulaire dégénérative et/ou de cardiomyopathie dilatée.

9. Composition vétérinaire selon la revendication 7 ou 8, pour son utilisation dans le traitement de sujets mammifères non humains en association avec des thérapies standards choisies parmi les antagonistes du récepteur AT-1 de l'angiotensine II, les digitaliques, les inotropes, les inodilatateurs, les diurétiques, les vasodilatateurs, les béta bloquants et/ou les antagonistes calciques.

10. Composition vétérinaire selon l'une quelconque des revendications 1-7, pour son utilisation dans traitement de sujets mammifères non humains atteints d'insuffisance cardiaque non décompensée, et **caractérisée en ce qu'**elle est administrée avant l'apparition d'oedèmes chez sujets mammifères non humains.

11. Composition vétérinaire selon l'une quelconque des revendications précédentes, **caractérisée en ce que** lesdits sujets atteints d'insuffisance cardiaque sont des animaux de compagnie choisis parmi les chiens, les chats, ou les chevaux.

12. Composition vétérinaire selon l'une quelconque des revendications précédentes, **caractérisée en ce qu'**elle se présente sous une forme administrable par voie orale, nasale, intradermique, cutanée ou parentérale.

13. Composition vétérinaire selon l'une quelconque des revendications précédentes, **caractérisée en ce qu'**elle se présente sous forme d'une solution liquide, sous forme d'une suspension, sous forme solide ou semi-solide, sous forme de poudres, de comprimés, de capsules, de granules, de dragées, de gélules, de sprays, de cachets, de pilules, de tablettes, de pâtes, d'implants ou de gels.

14. Kit à usage vétérinaire destiné au traitement de sujets mammifères non humains atteints d'insuffisance cardiaque, ayant au moins un compartiment comprenant une dose quotidienne thérapeutique efficace d'antagoniste de l'aldostérone comprise entre 2 et 5 mg/kg/jour, et de préférence d'environ 2 mg/kg/jour.

**15.** Kit à usage vétérinaire destiné au traitement de sujets mammifères non humains atteints d'insuffisance cardiaque, ayant au moins un compartiment comprenant une dose quotidienne thérapeutique efficace d'antagoniste de l'aldostérone comprise entre 2 et 5 mg/kg/jour, et de préférence d'environ 4 mg/kg/jour.

FIGURE 1

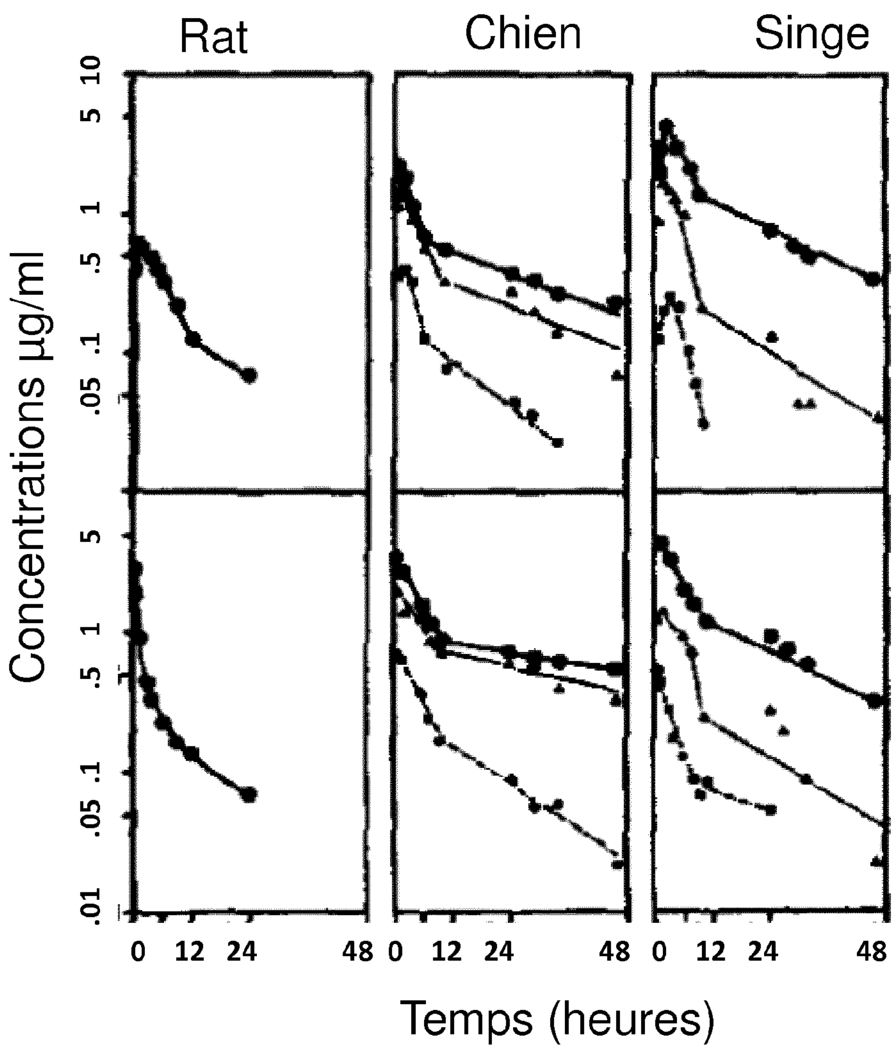
Rat
Chien
Singe
Concentrations µg/ml
10
5
1
.5
.1
.05
.01
0
12
24
48
Temps (heures)

FIGURE 2

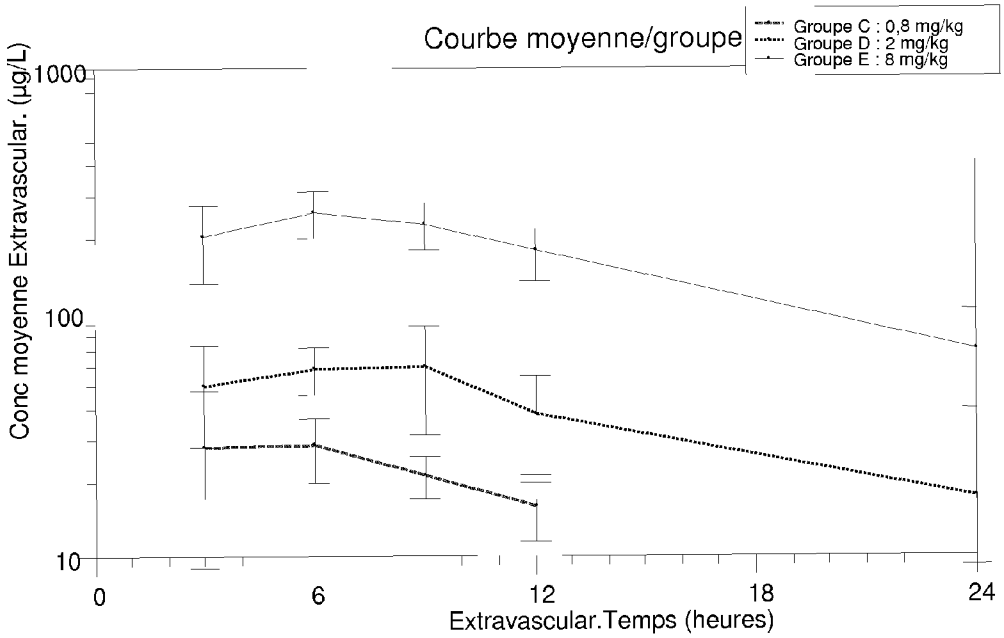
Courbe moyenne/groupe
Groupe C : 0,8 mg/kg
Groupe D : 2 mg/kg
Groupe E : 8 mg/kg
Conc moyenne Extravascular. (µg/L)
1000
100
10
0
6
12
18
24
Extravascular.Temps (heures)

FIGURE 3

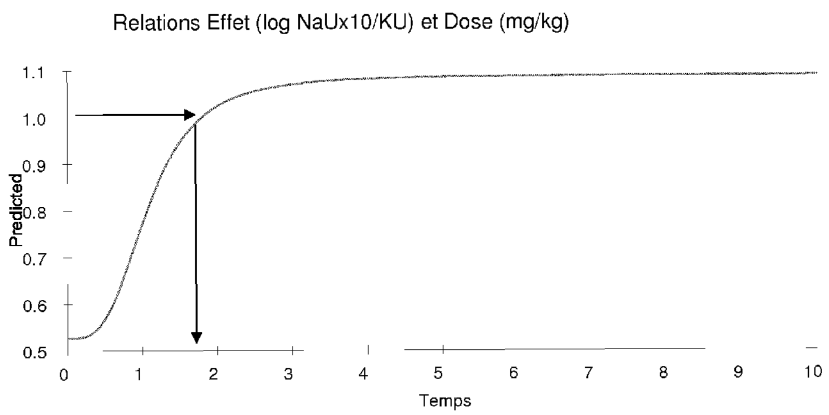


| Parametres | Valeur |
|---|---|
| Emax | 1,089 |
| EC50 | 1,077 |
| E0 | 0,5267 |
| Gamma | 3,275 |

FIGURE 4

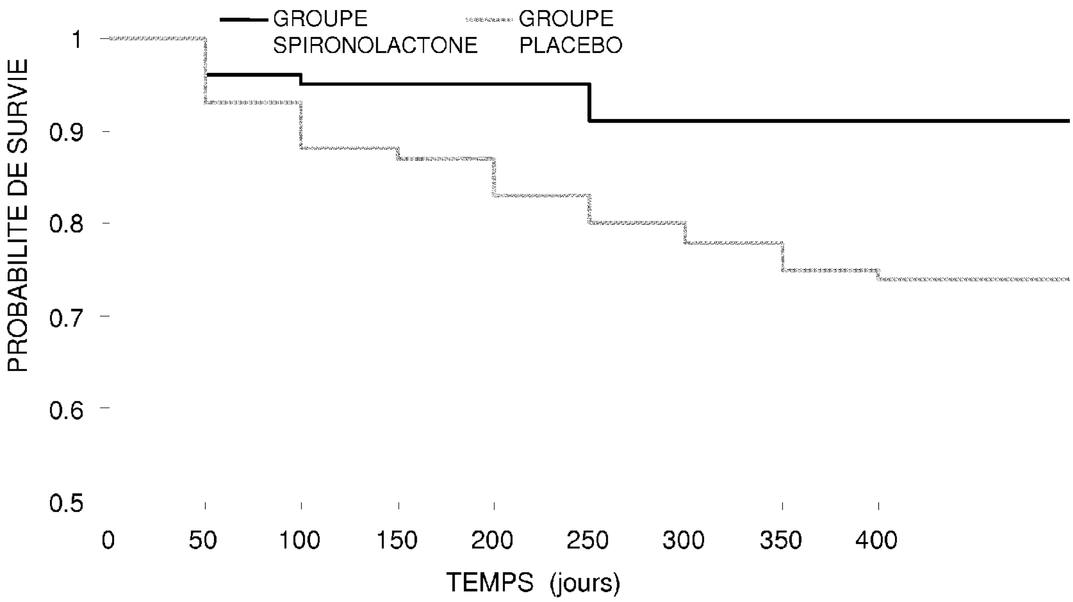
GROUPE SPIRONOLACTONE
GROUPE PLACEBO
PROBABILITE DE SURVIE
1
0.9
0.8
0.7
0.6
0.5
0
50
100
150
200
250
300
350
400
TEMPS (jours)

FIGURE 5

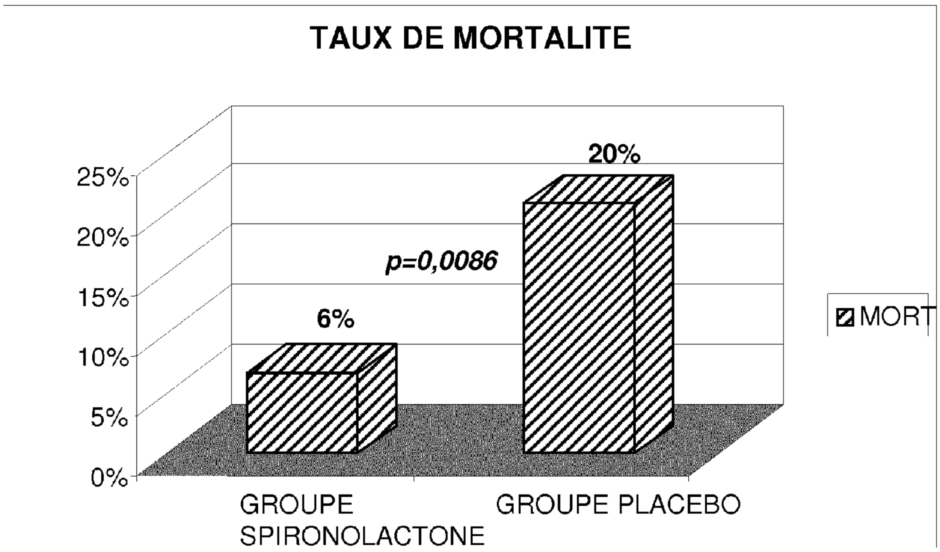
TAUX DE MORTALITE
25%
20%
15%
10%
5%
0%
20%
p=0,0086
6%
MORT
GROUPE SPIRONOLACTONE
GROUPE PLACEBO

FIGURE 6

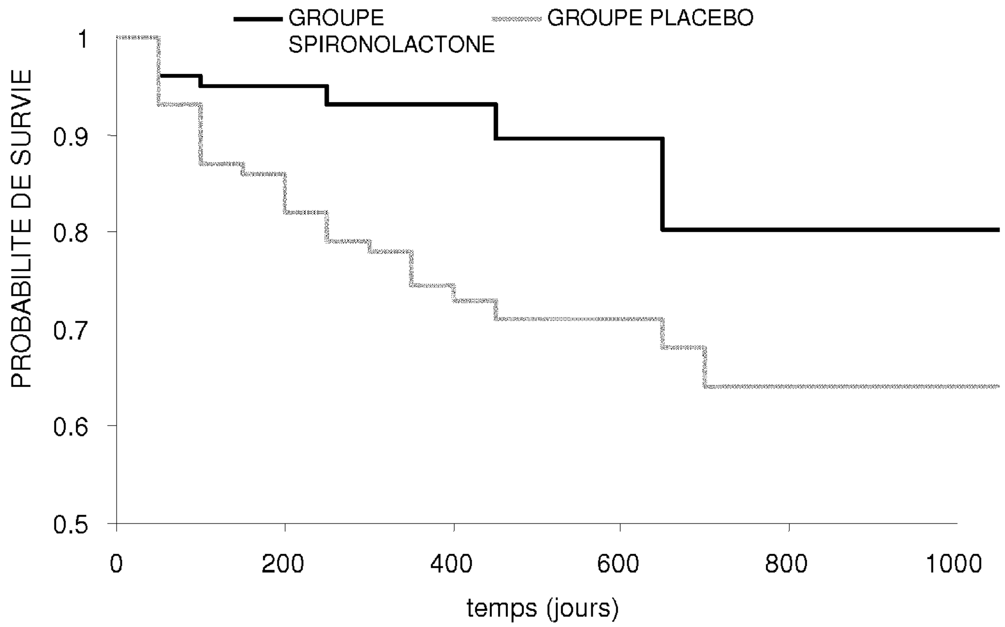
GROUPE SPIRONOLACTONE
GROUPE PLACEBO
PROBABILITE DE SURVIE
1
0.9
0.8
0.7
0.6
0.5
0
200
400
600
800
1000
temps (jours)

FIGURE 7

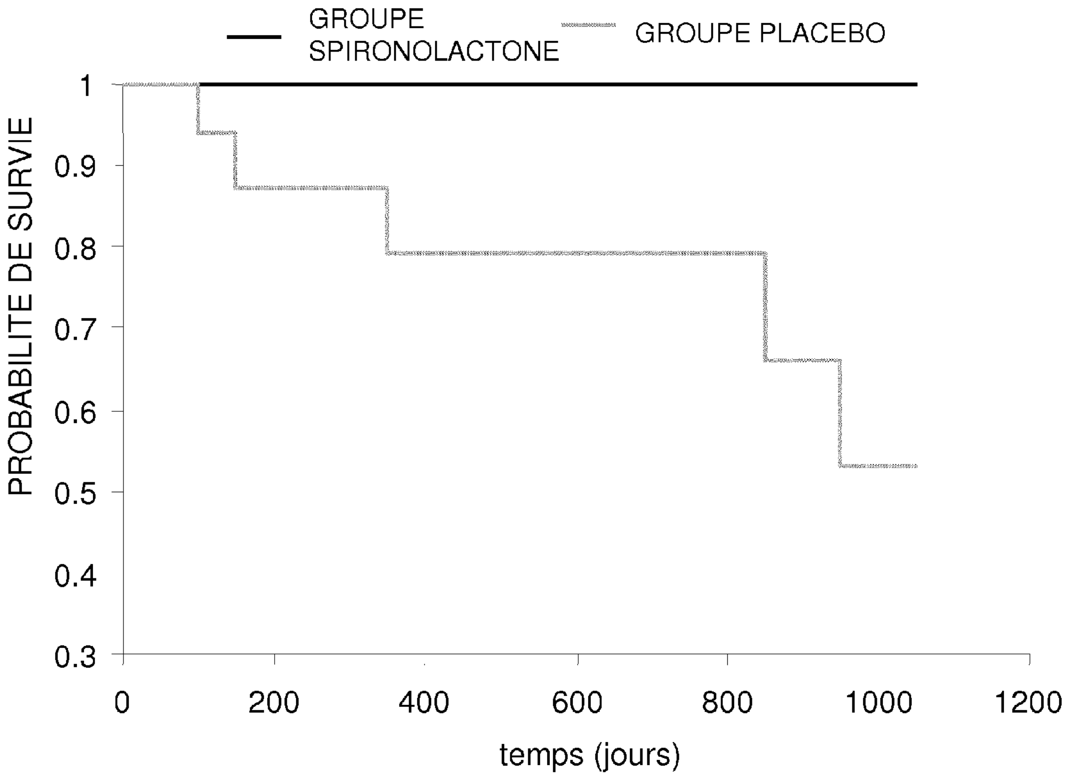
GROUPE SPIRONOLACTONE
GROUPE PLACEBO
PROBABILITE DE SURVIE
1
0.9
0.8
0.7
0.6
0.5
0.4
0.3
0
200
400
600
800
1000
1200
temps (jours)

FIGURE 8

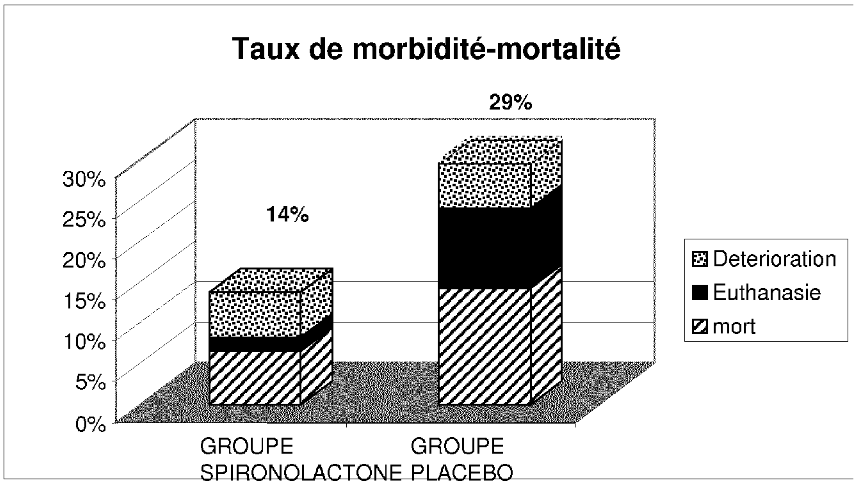
Taux de morbidité-mortalité
29%
14%
30%
25%
20%
15%
10%
5%
0%
GROUPE SPIRONOLACTONE
GROUPE PLACEBO
Deterioration
Euthanasie
mort

FIGURE 9

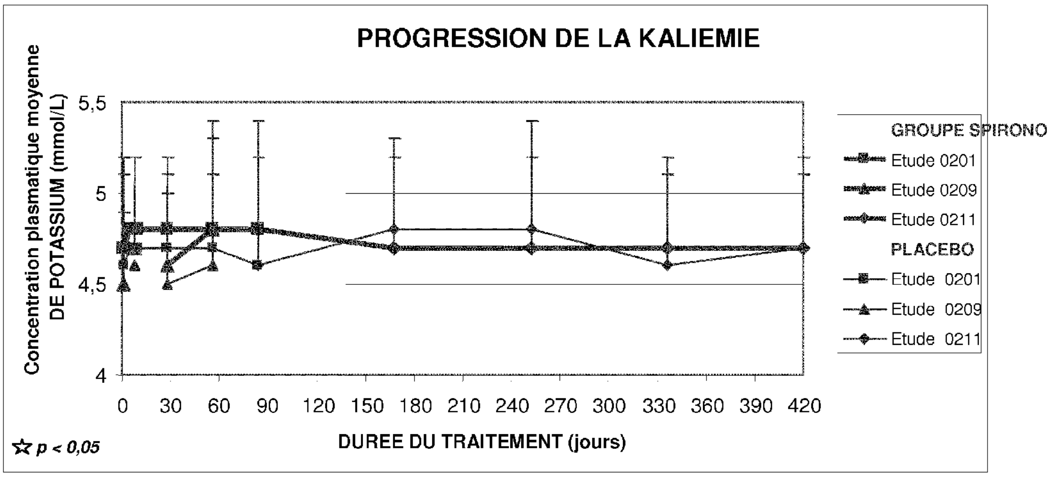
PROGRESSION DE LA KALIEMIE
Concentration plasmatique moyenne DE POTASSIUM (mmol/L)
5,5
5
4,5
4
0 30 60 90 120 150 180 210 240 270 300 330 360 390 420
DUREE DU TRAITEMENT (jours)
GROUPE SPIRONO
Etude 0201
Etude 0209
Etude 0211
PLACEBO
Etude 0201
Etude 0209
Etude 0211
☆ p < 0,05

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- WO 9624373 A
- EP 808172 B1
- US 4129564 A
- US 4559332 A
- US 4361563 A
- EP 008391 A
- EP 383449 A

### Littérature non-brevet citée dans la description


- **HOFMAN L.M et al.** *The Journal of Pharmacology and Experimental Therapeutics,* 1975, vol. 194, 450-456